# EUROPEAN PATENT SPECIFICATION

(11) **EP 3 681 918 B1**
(45) Date of publication and mention of the grant of the patent: **12.01.2022**
(21) Application number: 18759647.3
(22) Date of filing: 04.09.2018
(51) Int. Cl.: A61K 9/28, C08F 220/18, A61K 9/19, A61K 47/32, A61K 9/20, C09D 133/06, C09D 133/10, C08F 220/34, C08F 220/20

(54) **POLYMER AND DOSAGE FORM WITH SUSTAINED RELEASE PROPERTIES AND RESISTANCE AGAINST THE INFLUENCE OF ETHANOL**
POLYMER UND DOSIERUNGSFORM MIT VERZÖGERTER FREISETZUNG UND BESTÄNDIGKEIT GEGEN DEN EINFLUSS VON ETHANOL
POLYMÈRE ET FORME POSOLOGIQUE AYANT DES PROPRIÉTÉS DE LIBÉRATION PROLONGÉE ET UNE RÉSISTANCE CONTRE L'INFLUENCE DE L'ÉTHANOL

(30) Priority: 14.09.2017 EP 17191129; 09.03.2018 WO PCT/EP2018/055856
(43) Date of publication of application: 22.07.2020
(73) Proprietor: Evonik Operations GmbH, 45128 Essen (DE)
(72) Inventor: ENDRES, Thomas, Hoover, AL 35226 (US); MEIER, Christian, 64295 Darmstadt (DE); HERMES, Florian, 45721 Haltern am See (DE); DEL ROSARIO FERRAND, Jessica, 64859 Eppertshausen (DE); JUNG, Herbert, 63791 Karlstein (DE); EURICH, Thomas, 63452 Hanau (DE); SCHATTKA, Jan Hendrik, 64287 Darmstadt (DE)
(74) Representative: Evonik Patent Association
(86) International application number: PCT/EP2018/073652
(87) International publication number: WO 2019/052845

(56) References cited:
- WO-A1-2016/193034
- JP-A- 61 152 757

## Description

### Background

Alcohol resistant enteric formulations are known from
- WO 2009/036811: PH-dependent controlled release pharmaceutical composition for non-opioids with resistance against the influence of ethanol.
- WO 2009/036812: PH-dependent controlled release pharmaceutical opioid composition with resistance against the influence of ethanol.
- WO 20101034342: PH-dependent controlled release pharmaceutical opioid composition with resistance against the influence of ethanol.
- WO 20101034344: PH-dependent controlled release pharmaceutical composition for non-opioids with resistance against the influence of ethanol.
- WO 2012/171884: Gastric resistant pharmaceutical or nutraceutical composition with resistance against the influence of ethanol.
- A. Krieg, E. Arici, et al. (2014). "Toward pH-Responsive Coating Materials-High-Throughput Study of (Meth)acrylic Copolymers." ACS Combinatorial Science 16(8): 386-392.
- WO 2010/105672: Controlled release pharmaceutical composition with resistance against the influence of ethanol employing a coating comprising neutral vinyl polymers and excipients.
- WO 2010/105673: Controlled release pharmaceutical composition with resistance against the influence of ethanol employing a coating comprising a polymer mixture and excipients.
- WO 2015/121189: Pharmaceutical or nutraceutical composition with sustained release characteristic and with resistance against the influence of ethanol.
- WO 2014/151797: Extended release formulations resistant to alcohol dose dumping.
- WO 2016/193034A1: Pharmaceutical or nutraceutical composition with resistance against the influence of ethanol.
- Y. Rosiaux, C. Velghe, et al. (2013). "Ethanol-resistant ethylcellulose/guar gum coatings - Importance of formulation parameters." European Journal of Pharmaceutics and Biopharmaceutics 85(3, Part B): 1250-1258.
- Y. Rosiaux, C. Velghe, et al. (2014). "Mechanisms Controlling Theophylline Release from Ethanol-Resistant Coated Pellets." Pharmaceutical Research 31(3): 731-741.
- C. Y. Gujjar, B. C. Rallabandi, et al. (2015). "Development and Optimization of a Novel Prolonged Release Formulation to Resist Alcohol-Induced Dose Dumping." AAPS PharmSciTech: 1-8.
- WO 2012/171884 describes gastric resistant pharmaceutical or nutraceutical composition with resistance against the influence of ethanol. Disclosed is a (meth)acrylate copolymer comprising polymerized units of 10 to 40 % by weight of acrylic or methacrylic acid, 10 to 80 % by weight of a C₄- to C₁₈-alkyl ester of acrylic or methacrylic acid and optionally 0 to 60 % by weight of another vinylic monomer, whereby the release of the pharmaceutical or nutraceutical active ingredient is not more than 10 % under in-vitro conditions at pH 1.2 after 2 hours in medium according to USP with and without the addition of 20 % (w/w) ethanol.
- US 4737357 describes a method for producing a film-forming aqueous dispersions and coating agent for pharmaceuticals comprising a (meth)acrylate copolymer which is composed of free-radical polymerized methyl methacrylate, ethylacrylate, and 2-Trimethylammonium-ethyl-methacrylate-chloride.
- JP61152757A, WO2013/080964A1 and JP2016/180834A describe polymeric binders for paints, adhesives or soil conditioners or for color filter devices or photosensitive coloring compositions respectively.

### General definitions

Singular forms like "a", "an", "the" or "another" as used in the description or in the claims shall be understood as to include the plural of the defined subject within the given definition or limits as well if not stated explicitly otherwise.

Singular terms like "a C₁-C₁₂ alkylester of acrylic acid or of methacrylic acid" shall be understood as "C₁-C₁₂ alkylester of acrylic acid or C₁-C₁₂ alkylester of methacrylic acid" and shall also include one or more of these monomers and any mixtures thereof.

The terms "comprises" or "is comprising" shall understood as including the terms "essentially comprises" or "is essentially comprising" and "consisits" or "consiting of".

A "C₂-C₈ alkyl ester of acrylic acid or of methacrylic acid with a quaternary cationic group in the alkyl group" means that the alkyl group is substituted with a quaternary cationic group, preferably with a quaternary ammonium group. Examples for these well known monomers are 2-Trimethylammonium-ethyl-methacrylate-chloride (TMAEMC) or 2-Trimethylammonium-propyl-methacrylate-chloride (TMAPMC).

### Summary of the invention

Pharmaceutical or nutraceutical compositions are designed to release the active ingredient in a manner of reproducible release curves. This shall result in desirable and reliable blood level profiles which shall provide an optimal therapeutic effect. If the blood level concentrations are too low, the active ingredient will not cause a sufficient therapeutic effect. If the blood level concentrations are too high, this may cause toxic effects. In both cases non optimal blood level concentrations of an active ingredient can be dangerous for the patient and shall therefore be avoided. A problem exists in that the ideal ratios assumed for the release of active ingredient during the design of a pharmaceutical or nutraceutical composition can be altered by the general living habits, thoughtlessness or by addictive behaviour of the patients with respect to the use of ethanol or ethanol-containing drinks. In these cases, the pharmaceutical or nutraceutical form which is actually designed for an exclusively aqueous medium is additionally exposed to an ethanol-containing medium of greater or lesser strength. Since health authorities like for instance the US Food and Drug Administration (FDA) focus more and more on the ethanol problem, ethanol resistance may be an important registration requirement in the near future. The problem is severe especially for pharmaceutical compositions but of course also exist for nutraceutical compositions.

Since not all patients or customers are aware of the risk of simultaneous taking of a controlled release pharmaceutical or nutraceutical form and ethanol-containing drinks or do not follow or are not able to follow appropriate warnings, advice or recommendations, there is a demand for controlled release pharmaceutical or nutraceutical compositions, especially for gastric resistant or sustained release pharmaceutical or nutraceutical compositions, such that their mode of action is affected as little as possible by the presence of ethanol.

Conventional extended or sustained release pharmaceutical or nutraceutical compositions if coated or uncoated are usually not resistant to alcohol at all. Therefore one problem of the present invention was to provide extended or sustained release pharmaceutical or nutraceutical compositions which are resistant against the influence of ethanol.

Especially there is a problem for dosage forms with sustained release characteristic. These kinds of formulations are usually coated with water-insoluble polymers or copolymers onto a core comprising a pharmaceutical or nutraceutical active ingredient. The release of the biologically active ingredient, e.g. the pharmaceutical or nutraceutical active ingredient is sustained which means more or less constantly over the time (zero order release) and independent from the pH of the environment. The release of the pharmaceutical or nutraceutical active ingredient under in-vitro conditions after 2 hours at pH 1.2 in simulated gastric fluid according to USP (for instance USP 32) and subsequent change of the medium to buffered medium of pH 6.8 according to USP may for instance be in the range of 2 to 98, 30 to 90, 40 to 80 % in a total time, including the 2 hours of the pH 1.2 phase, of 4 to 12, 4 to 8 or 6 to 10 hours.

However the presence of ethanol in concentrations of 5, 10, 20 or 40 % (volume/volume) in the gastric fluid usually leads to an increase to the release rates already in the stomach. Thus an effective protection against the influence of ethanol should prevent such an undesired increase of pharmaceutical or nutraceutical active ingredient in the stomach but also in the intestine.

Thus the presence of ethanol in concentrations of 5, 10, 20 or 40 % (volume/volume) under in-vitro conditions after 2 hours at pH 1.2 in simulated gastric fluid according to USP (for instance USP 32) shall not severely influence the intended sustained or extended release rates at pH 1.2. Furthermore the presence of ethanol in concentrations of 5, 10, 20 or 40 % (volume/volume) under in-vitro conditions after 2 hours in pH 1.2 medium according to USP (for instance USP 32) and subsequent change of the medium to buffered medium of pH 6.8 according to USP without ethanol shall not severely influence the intended sustained or extended release rates at pH 1.2 and at pH 6.8.

Polymers like EUDRAGIT^{®} RL, EUDRAGIT^{®} RS or EUDRAGIT^{®} NM are widely used in pharmacy for the coating of sustained release dosage forms. However these polymers are not resistant against the influence of ethanol. Thus there is a need for new polymers with similar sustained release but also ethanol resistance properties.

The objects are solved as claimed.

### Details of the invention

### Polymer

The invention is concerned with a polymer, especially a (meth)acrylate copolymer, preferably a methacrylate copolymer, polymerized from a monomer mixture comprising the monomers
(a) 70 to 95% by weight of 2-Ethylhexyl methacrylate (EHMA) and Ethyl methacrylate (EMA) or 2-Ethylhexyl methacrylate (EHMA) and methyl methacrylate (MMA), most preferred 2-Ethylhexyl methacrylate (EHMA) and Ethyl methacrylate (EMA),
(b) 2.5 to 25, preferably 2.5 to 15% by weight of one or more C₂ to C₆ hydroxy-alkylesters of acrylic acid or methacrylic acid,
(c) 2.5 to 15% by weight of one or more C₂ to C₈ alkyl esters of acrylic acid or of methacrylic acid with a quaternary cationic group in the alkyl group.

The monomers (a), (b) and (c) may optionally add up to 100%.

Each "% by weight" range for each monomer may be combined with each weight-% range for another monomer.

Disclosed is also the use of the polymer for preparing a dosage form with a sustained release profile and resistance against the influence of ethanol and the dosage form itself.

### Monomers (a)

The monomers (a) are 2-Ethylhexyl methacrylate (EHMA) and Ethyl methacrylate (EMA) or 2-Ethylhexyl methacrylate (EHMA) and methyl methacrylate (MMA). Most preferred is the combination of 2-Ethylhexyl methacrylate (EHMA) and Ethyl methacrylate (EMA). 2-Ethylhexyl methacrylate (EHMA) and Ethyl methacrylate (EMA) or 2-Ethylhexyl methacrylate (EHMA) and methyl methacrylate (MMA) may be included as monomers (a1) and (a2). The ratio by weight of 2-Ethylhexyl methacrylate (EHMA) : Ethyl methacrylate (EMA) or of 2-Ethylhexyl methacrylate (EHMA): methyl methacrylate (MMA) may be preferably in the range from 5 : 1 to 1 : 1, from 4 : 1 to 1.5 : 1 or from 3.5 : 1 to 2 : 1.

### Monomers (b)

C₂ to Ce or C₂ to C₄ hydroxy-alkylesters of acrylic acid or methacrylic acid (b) are for instance 2-Hydroxyethyl methacrylate, 2-Hydroxypropyl methacrylate, 3-Hydroxypropyl methacrylate, 2,3-Dihydroxypropyl methacrylate, 2-Hydroxyethyl acrylate, 2-Hydroxypropyl acrylate, 3-Hydroxypropyl acrylate, 2,3-Dihydroxypropyl acrylate or any mixture thereof.

The C₂ to C₆ or C₂ to C₄ hydroxy-alkylesters of acrylic acid or methacrylic acid (b) are preferably selected from 2-Hydroxyethyl methacrylate (HEMA).

### Monomers (c)

The C₂ - C₈ alkyl ester of acrylic acid or of methacrylic acid with a quaternary cationic group, preferably a quaternary ammonium group, in the in the alkyl group (c) may be preferably 2-Trimethylammonium-ethyl-methacrylate-chloride (TMAEMC) or 2-Trimethylammonium-propyl-methacrylate-chloride (TMAPMC).

### Monomer (d)

The monomer mixture may further comprise less than 10, less than 8, less than 5 or less than 2 % by weight of a monomer (d) which is acrylic acid or methacrylic acid or both. The monomer mixture may further comprise 0.1 up to less than 10, 0.2 to 8, 0.5 to 5 or 0.8 to 2 % by weight of acrylic acid or methacrylic acid or both as a monomer (d). The monomers (a) to (c) and the monomer (d), acrylic acid and/or methacrylic acid, may add up to 100 %. The monomer mixture may as well comprise not any (0 %) acrylic acid or not any (0 %) methacrylic acid at all.

The monomer mixture may comprise monomers (a) to (c) and optionally monomer (d) and optionally also a monomer (d). The monomers (a) to (c) and also optionally monomer (d) may add up to 100 %.

Any single combination or mixture of the monomers (a1), (a2), (b), and (c) and optionally monomer (d) as disclosed above shall be herewith enclosed.

### Preferred embodiments

A preferred polymer may be polymerized from a monomer mixture comprising
(a1) 2-Ethylhexyl methacrylate,
(a2) Ethyl methacrylate
(b) 2-Hydroxyethyl methacrylate
(c) 2-Trimethylammonium-ethyl-methacrylate-chloride and optionally
(d) methacrylic acid and/or acrylic acid,
wherein the monomers (a1), (a2), (b) and (c) and optionally (d) may add up to 100 %.

In the preferred embodiments below generally the broader ranges given for one of the single monomers (a1), (a2), (b) or (c) or optionally (d) may be freely combined with the preferred narrower ranges of another monomer (a1), (a2), (b) or (c) or optionally (d). A skilled person can easily chose suitable weight percentages for the monomers (a1), (a2), (b) and (c) and optionally (d) from the disclosed ranges so that the monomer mixtures will add up to 100%.

In a preferred embodiment the polymer is polymerized from a monomer mixture comprising the monomers (a1), (a2), (b) and (c) and optionally a component (d) with
(a1) 40 - 80, preferably 47 - 74 % by weight 2-Ethylhexyl methacrylate
(a2) 15-30, preferably 18 - 25 % by weight Ethyl methacrylate
(b) 2.5 -15, preferably 4 -14 % by weight 2-Hydroxyethyl methacrylate
(c) 2.5 -15 preferably 4 - 14 % by weight 2-Trimethylammonium-ethyl-methacrylate-chloride and optionally
(d) 0 up to less than 10 % by weight acrylic acid or methacrylic acid,
wherein the monomers (a1), (a2), (b) and (c) and optionally (d) may add up to 100 %.

Monomer (d) may be optionally comprised from 0 up to less than 10, up to less than 8, up to less than 5 or up to less than 2 % or from 0.1 up to less than 10, 0.2 to 8, 0.5 to 5 or 0.8 to 2 % by weight. If no monomer (d) is comprised the monomers (a1), (a2), (b) and (c) may add up to 100 %.

In another preferred embodiment based on the inventive polymers 3 and 4 shown in the examples the polymer may be polymerized from a mixture of the monomers (a1), (a2), (b) and (c) with
(a1) 50 - 75, preferably 58 - 64 % by weight 2-Ethylhexyl methacrylate
(a2) 15-25, preferably 20 - 22 % by weight Ethyl methacrylate
(b) 7.5 -15, preferably 9-14 % by weight 2-Hydroxyethyl methacrylate
(c) 2.5 -10, preferably 4-6 % by weight 2-Trimethylammoniumethyl methacrylate-Chloride,
wherein the monomers (a1), (a2), (b) and (c) may add up to 100 %.

In another preferred embodiment based on the inventive polymers 8 and 9 shown in the examples the polymer may be polymerized from a monomer mixture comprising the monomers (a1), (a2), (b) and (c) with
(a1) 50 to 80, preferably 56 to 69 % by weight 2-Ethylhexyl methacrylate
(a2) 15 to 25, preferably 18 to 22 % by weight Ethyl methacrylate
(b) 2.5 to 10, preferably 4.5 to 8.5 % by weight 2-Hydroxyethyl methacrylate
(c) 2.5 to 15, preferably 8.5 to 13.5 % by weight 2-Trimethylammoniumethyl methacrylate-chloride
wherein the monomers (a1), (a2), (b) and (c) may add up to 100 %.

In a further preferred embodiment based on the inventive polymers 1 and 10 shown in the examples, the polymer types 1 and 10 may be mixed at ratios from 4:1 to 1:4, preferably at a ratios from 2 :1 to 1:2 to give a mixed coatings with intermediate release characteristics.

The polymer type 1 may be polymerized from a monomer mixture comprising the monomers (a1), (a2), (b) and (c) with
(a1) 63 to 75, preferably 65 to 71 % by weight 2-Ethylhexyl methacrylate,
(a2) 20 to 25, preferably 21 to 25 % by weight Ethyl methacrylate,
(b) 2.5 to 6, preferably 4 to 6 % by weight 2-Hydroxyethyl methacrylate and
(c) 2.5 to 6, preferably 4 to 6 % by weight 2-Trimethylammoniumethyl methacrylate-chloride,
wherein the monomers (a1), (a2), (b) and (c) may add up to 100 %..

The polymer type 10 may be polymerized from a monomer mixture comprising the monomers (a1), (a2), (b) and (c) with
(a1) 52.5 to 67.5, preferably 54.5 to 65.5 % by weight 2-Ethylhexyl methacrylate,
(a2) 17.5 to 22.5, preferably 17.5 to 22.5 % by weight Ethyl methacrylate,
(b) 5 to 10, preferably 6 to 9 % by weight 2-Hydroxyethyl methacrylate and
(c) 10 to 15, preferably 11 to 14 % by weight 2-Trimethylammoniumethyl methacrylate-chloride,
wherein the monomers (a1), (a2), (b) and (c) may add up to 100 %.

Preferably 0.1 to 0.5 % by weight, related to the total weight of the monomers, of a Chain-transfer agent, preferably Ethylhexylthioglycolat, may be added to the monomer mixtures disclosed above for controlling the molecular weight and weight distribution in the polymerization process.

### Minimum film forming temperature (MFFT)

Preferably the minimum film forming temperature (MFFT) of the polymer is 35 °C or lower, 30 °C or lower, 25 °C or lower, 20 °C or lower or 15 °C or lower.

Preferably the polymer shows the minimum film forming temperature (MFFT) of 5 to 35. 8 to 30, 9 to 25, 10 to 20 °C.

The MFFT may be determined according to the Standard of the International Organisation for Standardization DIN ISO 2115 with the exception of point 6.1 in that the maximum difference of the most distant metering points is set to 50 °C.

### Midpoint glass transition temperature (T_{mg})

Preferably the midpoint glass transition temperature (*T*_{mg}) of the polymer may be in the range of 0 to 50, 5 to 40, 8 to 25 or 8 to 20 °C.

DSC measurement of the dry polymer substance was conducted according to DIN EN ISO 11357-2 with a heating rate of 20 °C/min. The midpoint glass transition temperature *T*_{mg} was determined by half step height method as described in section 10.1.2 of DIN EN ISO 11357-2.

### Molecular weight Mw - Polydispersity Index

Preferably the weight average molecular weight M_{w} of the polymer as disclosed is from 10.000 to 200.000, 50.000 to 150.000, 60.000 to 140.000 or 70.000 to 130.000, 80.000 to 120.000, 90.000 to 110.000 Dalton.

The polydispersity index may be determined by calculation of the M_{w}/Mₙ ratio (weight average molecular weight/number average molecular weight (determined by GPC)). The polydispersity index of the inventive polymer may be in the range from 1.2 to 4.0, 1.3 to 3.0, 1.5 to 2.5 or from 1.6 to 2.3.

Gel permeation chromatography (GPC) is used to determine the number- and weight-average molecular weights (Mₙ, M_{w}) and the polydispersity (D) of the inventive polymers as disclosed according to DIN 55672-1. Equipment consisted of four PSS SDV columns (Mainz, Germany) plus pre-column of the same type, a column oven operating at 35 °C, an Agilent (Series 1100, Santa Clara, USA) pump plus RI-detector of the same series. A 0.02 M solution of 2-(Diethylamino)ethylamine (DEAEA) in Tetrahydrofuran (THF) was used as eluent at a flow rate of 1 mL/min. Samples were dissolved in the eluent at concentrations of 2 mg/mL. For each measurement 100 µL polymer solution was injected.. The values for Mn and Mw were calculated based on calibration curves generated by Poly(methyl methacrylate) standards.

EUDRAGIT^{®} reference samples were measured using the eluent N,N-dimethylacetamide (DMAc). Method for EUDRAGIT^{®} RL/RS is described in more detail by Adler M. et al. (e-Polymers, ISSN (Online) 1618-7229, ISSN (Print) 2197-4586, DOI: https://doi.org/10.1515/epoly.2005.5.1.602). Method for EUDRAGIT^{®} NM is described in more detail by Adler M. et al. (e-Polymers, ISSN (Online) 1618-7229, ISSN (Print) 2197-4586, DOI: https://doi.org/10.1515/epoly.2004.4.1.608).

### Process for preparing the polymers

A process for preparing the polymers disclosed herein may comprise the polymerization from the monomer mixture in the presence of a polymerization initiator and optionally a chain transfer agent by bulk polymerization, suspension polymerization or emulsion polymerization.

The polymer is preferably a (meth)acrylate copolymer (composed only from (meth)acrylate monomers) and may be produced by radical polymerisation of the monomers in the presence of polymerisation initiators such as ammonium-peroxodisulfate.

A Chain transfer agent may be added to improve the process stability and reproducibility of the molecular weight (Mw). However the Chain-transfer agent may be omitted in many cases, without affecting the properties according to the invention.

Preparation methods for the polymer are known to the expert in the field. Typically emulsion polymerization, solution polymerization or bulk polymerization will be applied; the preferred preparation of the polymer is by emulsion polymerization.

If emulsion polymerization is used, the operation may advantageously be carried out by the monomer emulsion feed process or the monomer feed process, respectively. For this, water is heated to the reaction temperature in the polymerization reactor. Surfactants and/or initiators may be added at this stage. Then, depending on the mode of operation, the monomer, a monomer mixture or an emulsion of either are fed to the reactor. This dosed liquid may contain initiators and/or surfactants or the initiator and/or the surfactant may be dosed in parallel.

Alternatively, all monomers can be charged into the reactor, before adding the initiator. This method is often referred to as batch process.

It is also possible to do a combination of both processes, by polymerizing a part of the monomers in the manner of a batch process, and feeding the other part afterwards.

As known to the expert in the field, the type of process and mode of operation can be chosen, to achieve the desired particle size, sufficient dispersion stability, a stable production process and so on.

### Emulsifiers

Emulsifiers which may be used are especially anionic and non-ionic surfactants. The amount of emulsifier used is generally not more than 5% by weight, preferably in the range of 0.1 to 4 % by weight, based on weight of the monomer mixture.

Typical emulsifiers are for example alkyl sulfates (e.g. sodium dodecyl sulfate), alkyl ether sulfates, dioctyl sodium sulfosuccinate, polysorbates (e.g. polyoxyethylene (20) sorbitan monooleate), nonylphenol ethoxylates (nonoxynol-9) and others.

### Polymerization Initiators

Beside those initiators conventionally used in emulsion polymerization (e.g. per-compounds, such as ammonium peroxodisulfate (APS)) redox systems, such as sodium disulphite-APS-iron can be applied. Also water soluble azo-initiators may be applied and/or a mixture of initiators can be used. The amount of polymerization initiator may be around 0.005 to 0.5, 0.05 to 0.2, 0.01 to 0.1 % by weight, based on total weight of the (meth)acrylate monomers.

### Chain-transfer agents

Chain-transfer agents are well known to the skilled person and used for controlling the molecular weight and weight distribution in a polymerization process.

A Chain-transfer agent may be added to the monomer mixture before or during the polymerization. Up to 5, up to 4, up to 3, up to 2, up to 1 % by weight or 0.05 to 5, 0.1 to 4, 0.2 to 3, 0.25 to 2, 0.1 to 1, 0.05 to 0.5, 0.1 to 0.4 % by weight of a Chain transfer agent, calculated on the total weight (100%) of the monomers, may be added to the monomer mixture. It is also possible to add not any Chain-transfer agent at all (0 %).

A suitable chain-transfer agent may be 2-Ethylhexylthioglycolat (TGEH) or n-Butylmercaptan, n-Dodecylmercaptan or 2-Mercaptoethanol or any mixtures thereof.

### Polymerization temperature

A suitable polymerization temperature may be in the range of 25 to 120, 30 to 100, 50 to 95 °C.

The polymerization temperature may depend on the initiators within certain limits. For example, if APS is used it is advantageous to operate in the range from 60 to 90° C; if redox systems are used it is also possible to polymerize at lower temperatures, for example in the range of 25 to 45 °C, for instance at 30° C.

The average particle size of the polymer particles produced in the emulsion polymerization may range from 10 to 1000, 20 to 500 or 50 to 250 nm. The average particle size of the polymer particles may be determined by methods well known to a skilled person for instance by the method of laser diffraction. The particle size may be determined by laser diffraction, using a Mastersizer 2000 (Malvern). The values can be indicated as particle radius rMS [nm], which is half of the median of the volume based particle size distribution d(v,50).

The obtained dispersion can directly be used to prepare the coating suspension, or - in rare cases - be used as coating suspension without even adding further ingredients.

The dispersion can also be dried, preferably by spray drying, freeze drying or coaguation. Thus a solid can be obtained, which offers certain advantages with regard to handling and logistics.

The dried polymer may then be transferred into a coating suspension by redispersing the solid in water, e.g. (where required) by the use of a high shear mixer.

The dried polymer may also be dissolved in a solvent, e.g. an organic solvent, to prepare a coating solution.

If coating with coating solutions is preferred, the preparation of the polymer by solution polymerization or bulk polymerization may be a good option, too.

### Sustained or extended release pharmaceutical or nutraceutical composition

The composition as disclosed herein is preferably a pharmaceutical or nutraceutical composition, preferably a sustained release or extended release pharmaceutical or nutraceutical composition.

The sustained or extended release of the pharmaceutical or nutraceutical active ingredient may be defined in that the active ingredient release under in-vitro conditions after 2 hours at pH 1.2 in simulated gastric fluid according to USP (for instance USP 32) and subsequent change of the medium to buffered medium of pH 6.8 according to USP may be for instance in the range of 2 to 98, 30 to 90, 40 to 80 % in a total time of 4 to 12 or 4 to 8 or 6 to 10 hours, including the 2 hours of the pH 1.2 phase.

### Ethanol resistant composition

The composition as disclosed herein is an ethanol (EtOH) resistant composition, preferably an ethanol (EtOH) resistant pharmaceutical or nutraceutical composition.

Ethanol resistant shall mean that the release of a biologically active ingredient, preferably a pharmaceutical or nutraceutical active ingredient, under in-vitro conditions at pH 1.2 for 2 hours in simulated gastric fluid according to USP and subsequent buffer pH 6.8 without the addition of ethanol does not differ by more than plus/minus 20, preferably plus/minus 10 % (absolute percentage) in the same media but with the addition of 5, 10, 20 or 40 % (w/w) ethanol in the pH 1.2 medium only.

To give an example if the release rate of the pharmaceutical or nutraceutical active ingredient is in the medium without ethanol for instance 60 % then the active ingredient release in the same medium with ethanol shall be in the range from 40 to 80 % (+/- 20 % deviation).

Ethanol resistant dosage forms as defined herein are formulations with release kinetics in pH 1.2 medium and subsequent pH 6.8 medium not significantly affected by the presence of ethanol in a pH 1.2 medium. Ethanol resistance may be an important registration requirement in the near future. Conventional pharmaceutical compositions if coated or uncoated are usually not resistant to alcohol at all. An ethanol resistant formulation is sometimes also called a rugged formulation.

Resistance against the influence of ethanol (ethanol resistant dosage form) may be defined in that the release profile determined under in-vitro conditions at pH 1.2 and/or at pH 6.8 in a buffered medium according to USP with the addition of 40 % (w/w) ethanol is not accelerated by more than 20 %, preferably by not more than 10 %, and not delayed by more than 20 %, preferably by not more than 10 %, under the influence of the 40 % ethanol containing medium in comparison to a release profile determined in the same medium without ethanol. Generally an acceleration of a release profile is more critical than a delay. Therefore, the upper limit for an acceleration of the release profile is preferably not more than 10 %, more preferably not more than 5 %, even more preferably there is no acceleration of the release profile at all.

Depending on the certain dosage form the applicable conditions of the USP test may vary for instance if the paddle or basket method has to be used or the stirring has to be 50, 100 or 150 rpm. For the determination of the ethanol resistance it does not matter which USP test is applied for the certain pharmaceutical composition as long as it is the relevant test for the certain pharmaceutical (or nutraceutical) composition and the test conditions with and without ethanol are the same.

Resistance against the influence of ethanol in the sense of the present invention shall be tested in a relevant period of the release of the active ingredient, where meaningful results can be expected. The period which is meaningfully chosen is from or between 10 to 80 % of the total dosage release in the medium without ethanol. In this period the resistance against the influence of ethanol shall be determined at a number n of at least n=3, but preferably more than 3, for instance n=4, 5, 6, 7, 8, 9, 10, 11 or 12 uniformly distributed test points. The number of meaningfully chosen test points depends on the total time period of the release profile from or between 10 to 80 % of the total dosage release. The longer the time period the more uniformly distributed test points can be chosen meaningful. The first test point should be the first full hour or half hour time point at or after the 10 % release point. The last test point should be at the last full hour or half hour time point at or before the 80 % release point. The other test point or test points should be in the middle (n=3) or uniformly distributed (n>3) at full hour or half hour time points at or in between the 10 and 80 % release phase. The percentage of acceleration or delay is calculated by the arithmetic mean (arithmetic average) of the n values to give the arithmetic mean release.

The term "and/or" in "under in-vitro conditions at pH 1.2 and/or at pH 6.8" means that there may be different meaningful conditions for different pharmaceutical (or nutraceutical) compositions. Resistance against the influence of ethanol shall be determined only in a relevant period of the release of the active ingredient.

Sustained release pharmaceutical compositions have periods of the release of the active ingredient for instance from 6 to 12 or even more hours, with usually more than 10 % release within the first two hours at pH 1.2. In this case it is meaningful to test under in-vitro conditions at pH 1.2 and at pH 6.8.

The percentages of acceleration or delay under the influence of the 5, 10, 20 or 40 % ethanol containing pH 1.2 medium are calculated by subtraction of corresponding single release values and the calculation of the arithmetic average thereof. The n release values taken from the media (pH 1.2 and subsequent pH 6.8) with ethanol in the pH 1.2 medium are subtracted by the corresponding n release values from the media without ethanol in the pH 1.2 medium and the arithmetic average of the differences is calculated. A positive result stands for an acceleration of the release; a negative result stands for a delayed release.

A dosage form, which fulfils these conditions can be considered to be resistant against critically accelerated release or delay of the active compound by thoughtlessness or by addictive behaviour of the patients with respect to the use of ethanol or ethanol-containing drinks. This situation relates essentially to the simultaneous or subsequent consumption of an alcoholic drink together with the taking of the controlled release pharmaceutical form, such that the pharmaceutical form is exposed to a strong ethanol-containing medium in the stomach or intestine.

However, the purpose of the present invention is expressively not to stimulate, to promote or to make possible the consumption of ethanol-containing drinks together with delayed-release pharmaceutical forms, but to alleviate or to avoid the possibly fatal consequences of intentional or inadvertent misuse or abuse.

### Calculation example 1:

If the arithmetic average calculated from the active ingredient release in the medium with ethanol and without ethanol is 8 % (= plus 8 %), then there is an acceleration caused by the influence of ethanol of 8 %. In this case the controlled release pharmaceutical composition is regarded to be resistant against the influence of ethanol because it is within the limit of not more than 20 % acceleration.

### Calculation example 2:

If the arithmetic average calculated from the active ingredient release in the medium with ethanol and without ethanol is minus 23 % (- 23%), then there is a delay caused by the influence of ethanol of 23 %. In this case the controlled release pharmaceutical composition is not regarded to be resistant against the influence of ethanol because it is out of the limit of not more than 20 % delay.

### Biologically active ingredients

The biologically active ingredient may be preferably a pharmaceutical active ingredient and/or a nutraceutical active ingredient.

### Pharmaceutical active ingredients

The invention is preferably useful for sustained release formulated pharmaceutical dosage forms.

Therapeutical and chemical classes of active ingredients used in sustained release formulated coated pharmaceutical dosage forms are for instance analgetics, antibiotics or anti-infectives, antibodies, antiepileptics, antigens from plants, antirheumatics, betablocker, benzimidazole derivatives, beta-blocker, cardiovascular drugs, chemotherapeutics, CNS drugs, digitalis glycosides, gastrointestinal drugs, e.g. proton pump inhibitors, enzymes, hormons, liquid or solid natural extracts, oligonucleotides, peptidhormon proteins, therapeutical bacteria, peptides, proteins (metal)salt f.e. aspartates, chlorides, orthates, urology drugs, vaccines

Further examples of drugs for sustained controlled release may be: acamprosat, aescin, amylase, acetylsalicylic acid, adrenalin, 5-amino salicylic acid, aureomycin, bacitracin, balsalazine, beta carotene, bicalutamid bisacodyl, bromelain, bromelain, budesonide, calcitonin, carbamacipine, carboplatin, cephalosporins, cetrorelix, clarithromycin,chloromycetin, cimetidine, cisapride, cladribine, clorazepate, cromalyn, 1-deaminocysteine-8-D-arginine-vasopressin, deramciclane, detirelix, dexlansoprazole, diclofenac, didanosine, digitoxin and other digitalis glycosides, dihydrostreptomycin, dimethicone, divalproex, drospirenone,duloxetine, enzymes, erythromycin, esomeprazole, estrogens, etoposide, famotidine, fluorides, garlic oil, glucagon, granulocyte colony stimulating factor (G-CSF), heparin, hydrocortisone, human growth hormon (hGH), ibuprofen, ilaprazole, insulin, Interferon, Interleukin, Intron A, ketoprofen, lansoprazole, leuprolidacetat lipase, lipoic acid, lithium, kinin, memantine, mesalazine, methenamine, methylphenidate, milameline, minerals, minoprazole, naproxen, natamycin, nitrofurantion, novobiocin, , olsalazine, omeprazole, orothates, pancreatin, pantoprazole, parathyroid hormone, paroxetine, penicillin, perprazol, pindolol, polymyxin, potassium, pravastatin, prednisone, preglumetacin progabide, pro-somatostatin, protease, quinapril, rabeprazole, ranitidine, ranolazine, reboxetine, rutosid, somatostatin streptomycin, subtilin, sulfasalazine, sulphanilamide, tamsulosin, tenatoprazole, thrypsine, valproic acid, vasopressin, vitamins, zinc, including their salts, derivatives, polymorphs, isomorphs, or any kinds of mixtures or combinations thereof.

Further examples for pharmaceutical active ingredients may be caffeine citrate, metoprolol succinate and theophylline (as used in the examples).

### Nutraceutical active ingredients

Nutraceuticals are well known to the skilled person. Nutraceuticals are often defined as extracts of foods claimed to have medical effects on human health. Thus, nutraceutical active ingredients may display pharmaceutical activities as well: Examples for nutraceutical active ingredients may be resveratrol from grape products as an antioxidant, soluble dietary fiber products, such as psyllium seed husk for reducing hypercholesterolemia, broccoli (sulphane) as a cancer preservative, and soy or clover (isoflavonoids) to improve arterial health. Thus it is clear that many substances listed as nutraceuticals may also be used as pharmaceutical active ingredients.

Depending on the territory, the specific application, the local authority legislation and classification, the same substance may be listed as a pharmaceutical or as a nutraceutical active ingredient respectively as a pharmaceutical or a nutraceutical composition or even both. Thus it is evident to a skilled person that there is a broad overlap between the terms pharmaceutical or a nutraceutical active ingredient respectively a pharmaceutical or a nutraceutical composition.

The invention is preferably useful for nutraceutical dosage forms.

Nutraceuticals or nutraceutical active ingredients are sometimes defined as extracts of foods claimed to have medical effects on human health.

Nutraceuticals or nutraceutical active ingredients may also include probiotics and prebiotics. Probiotics are living microorganisms believed to support human or animal health when consumed, for example certain strains of the genera *Lactobacillus* or *Bifidobacterium.* Prebiotics are nutraceuticals or nutraceutical active ingredients that induce or promote the growth or activity of beneficial microorganisms in the human or animal intestine.

The nutraceutical active ingredient may be usually contained in a medical format such as capsule, tablet or powder in a prescribed dose. Examples for nutraceuticals are resveratrol from grape products or pro-anthocyanines from blueberries as antioxidants, soluble dietary fiber products, such as psyllium seed husk for reducing hypercholesterolemia, broccoli (sulphane) as a cancer preservative, and soy or clover (isoflavonoids) to improve arterial health. Other nutraceuticals examples are flavonoids, antioxidants, alpha-linoleic acid from flax seed, beta-carotene from marigold petals or antocyanins from berries. Sometimes the expression neutraceuticals or nutriceuticals are used as synonyms for nutraceuticals.

### Dosage form

Disclosed is a dosage form, preferably a pharmaceutical or nutraceutical dosage from, comprising a core, comprising a biologically active ingredient, and a coating layer onto the core, wherein the coating layer is comprising a polymer according to one or more of Claim 1 to 9 or a polymer, polymerized from a monomer mixture comprising the monomers
(a) 70 to 95% by weight of a C₁ - C₁₂ alkylester of acrylic acid or of methacrylic acid,
(b) 2.5 to 25, preferably 2.5 to 15 % by weight of a C₂ - C₆ hydroxy-alkylester of acrylic acid or methacrylic acid
(c) 2.5 to 15% by weight of a C₂ - C₈ alkyl ester of acrylic acid or of methacrylic acid with a quaternary cationic group in the alkyl group.

The monomers (a), (b) and (c) may optionally add up to 100%.

### Monomers (a)

C₁ to C₁₂ alkyl esters of acrylic acid or of methacrylic acid (a) are for instance methyl acrylate, ethyl acrylate, propyl acrylate, iso- propyl acrylate, butyl acrylate, pentyl acrylate, hexyl acrylate, heptyl acrylate, octyl acrylate, nonyl acrylate, decyl acrylate or dodecyl acrylate (lauryl acrylate), methyl methacrylate, ethyl methacrylate, propyl methacrylate, iso-propyl methacrylate, butyl methacrylate, pentyl methacrylate, hexyl methacrylate, heptyl methacrylate, octyl methacrylate, nonyl methacrylate, decyl methacrylate or lauryl methacrylate or any mixtures thereof.

The monomers (a) are preferably 2-Ethylhexyl methacrylate (EHMA) and Ethyl methacrylate (EMA) or 2-Ethylhexyl methacrylate (EHMA) and methyl methacrylate (MMA). Most preferred is the combination of 2-Ethylhexyl methacrylate (EHMA) and Ethyl methacrylate (EMA). 2-Ethylhexyl methacrylate (EHMA) and Ethyl methacrylate (EMA) or 2-Ethylhexyl methacrylate (EHMA) and methyl methacrylate (MMA) may be included as monomers (a1) and (a2). The ratio by weight of 2-Ethylhexyl methacrylate (EHMA): Ethyl methacrylate (EMA) or of 2-Ethylhexyl methacrylate (EHMA): methyl methacrylate (MMA) may be preferably in the range from 5 : 1 to 1 : 1, from 4 : 1 to 1.5 : 1 or from 3.5 : 1 to 2 : 1.

### Monomers (b)

C₂ to C₆ or C₂ to C₄ hydroxy-alkylesters of acrylic acid or methacrylic acid (b) are for instance 2-Hydroxyethyl methacrylate, 2-Hydroxypropyl methacrylate, 3-Hydroxypropyl methacrylate, 2,3-Dihydroxypropyl methacrylate, 2-Hydroxyethyl acrylate, 2-Hydroxypropyl acrylate, 3-Hydroxypropyl acrylate, 2,3-Dihydroxypropyl acrylate or any mixture thereof.

The C₂ to C₆ or C₂ to C₄ hydroxy-alkylesters of acrylic acid or methacrylic acid (b) are preferably selected from 2-Hydroxyethyl methacrylate (HEMA).

### Monomers (c)

The C₂ - C₈ alkyl ester of acrylic acid or of methacrylic acid with a quaternary cationic group, preferably a quaternary ammonium group, in the in the alkyl group (c) may be preferably 2-Trimethylammonium-ethyl-methacrylate-chloride (TMAEMC) or 2-Trimethylammonium-propyl-methacrylate-chloride (TMAPMC).

### Monomer (d)

The monomer mixture may further comprise less than 10, less than 8, less than 5 or less than 2 % by weight of a monomer (d) which is acrylic acid or methacrylic acid or both. The monomer mixture may further comprise 0.1 up to less than 10, 0.2 to 8, 0.5 to 5 or 0.8 to 2 % by weight of acrylic acid or methacrylic acid or both as a monomer (d). The monomers (a) to (c) and the monomer (d), acrylic acid and/or methacrylic acid, may add up to 100 %. The monomer mixture may as well comprise not any (0 %) acrylic acid or not any (0 %) methacrylic acid at all.

The monomer mixture may comprise monomers (a) to (c) and optionally also a monomer (d). The monomers (a) to (c) and also optional monomer (d) may add up to 100 %.

### Minimum film forming temperature (MFFT)

Preferably the minimum film forming temperature (MFFT) of the polymer is 35 °C or lower, 30 °C or lower, 25 °C or lower, 20 °C or lower or 15 °C or lower.

Preferably the polymer shows the minimum film forming temperature (MFFT) of 5 - 35. 8 - 30, 9 - 25, 10-20 °C.

The MFFT may be determined according to the Standard of the International Organisation for Standardization DIN ISO 2115 with the exception of point 6.1 in that the maximum difference of the most distant metering points is set to 50 °C.

### Midpoint glass transition temperature (T_{mg})

Preferably the midpoint glass transition temperature (*T*_{mg}) of the polymer comprised in the dosage form may be in the range of 0 to 50, 5 to 40, 8 to 25 or 8 to 20 °C.

DSC measurement of the dry polymer substance was conducted according to DIN EN ISO 11357-2 with a heating rate of 20 °C/min. The midpoint glass transition temperature *T*_{mg} was determined by half step height method as described in section 10.1.2 of DIN EN ISO 11357-2.

### Molecular weight Mw - Polydispersity Index

Preferably the weight average molecular weight M_{w} of the polymer comprised in the dosage form is from 10.000 to 200.000, 50.000 to 150.000, 60.000 to 140.000 or 70.000 to 130.000, 80.000 to 120.000, 90.000 to 110.000 Dalton.

The polydispersity index may be determined by calculation of the M_{w}/Mₙ ratio (weight average molecular weight/number average molecular weight (determined by GPC)). The polydispersity index of the inventive polymer may be in the range from.̅ 1.2 to 4.0, 1.3 to 3.0, 1.5 - 2.5 or from 1.6 to 2.3.

Gel permeation chromatography (GPC) may be used to determine the number- and weight-average molecular weights (Mₙ, M_{w}) and the polydispersity (D) of the polymers as disclosed according to DIN 55672-1. Equipment consisted of four PSS SDV columns (Mainz, Germany) plus pre-column of the same type, a column oven operating at 35 °C, an Agilent (Series 1100, Santa Clara, USA) pump plus RI-detector of the same series. A 0.02 M solution of 2-(Diethylamino)ethylamine (DEAEA) in Tetrahydrofuran (THF) was used as eluent at a flow rate of 1 mL/min. Samples were dissolved in the eluent at concentrations of 2 mg/mL. For each measurement 100 µL polymer solution was injected.. The values for Mn and Mw were calculated based on calibration curves generated by Poly(methyl methacrylate) standards.

The dosage form comprises a core which comprises a biologically active ingredient. Depending on the intended use in the pharmaceutical and/or in the nutraceutical field, the biologically active ingredient may be a pharmaceutical active ingredient and/or a nutraceutical active ingredient. A pharmaceutical active ingredient or a nutraceutical active ingredient is a biologically active ingredient comprised in an oral dosage form with an intended application or use in the field of pharmaceuticals and/or nutraceuticals.

The intention of the application of the dosage form in the pharmaceutical field is usually the therapy of diseases of man or animals. The intention of the application of the dosage form in the nutraceutical field is usually the prevention of diseases and general support of vitality and health of man or animals.

The dosage form is preferably an oral dosage form and depending on the intended use a pharmaceutical or nutraceutical dosage form. The dosage form comprises, comprises essentially or is consisting of a core and a coating layer. The core comprises a biologically active ingredient. The coating is located directly or indirectly onto the core and comprises the polymer as disclosed herein and optionally excipients, such as pharmaceutical or nutraceutical acceptable excipients. The dosage form, respectively the pharmaceutical or nutraceutical dosage form is intended to be used as an oral dosage form in the field of pharmaceuticals and/or nutraceuticals.

### Core

The core may be a pellet, a granule, a tablet or a capsule. The core is coated with a coating layer comprising the polymer as disclosed and optionally excipients, preferably pharmaceutical or nutraceutical acceptable excipients. The core may be an active ingredient containing tablet, a pellet containing compressed tablet, a mini tablet or a capsule, which may be filled with pellets.

The core may comprise an uncoated pellet, a neutral carrier pellet, for instance a sugar sphere or non-pareilles, on top of which the biologically active ingredient is bound in a binder, such as lactose or polyvinyl pyrrolidone. The layer with the biologically active ingredient is considered herein as part of the core. The core may as well comprise an uncoated pellet consisting of a crystallized biologically active ingredient.

The core may comprise 1 to 100, 2 to 90, 5 to 85, 10 to 70, 15 to 50 % by weight of the biologically active ingredient. The core may comprise 0 to 99, 10 to 98, 15 to 95, 30 to 90 or 50 to 85 % by weight of excipients, preferably pharmaceutical or nutraceutical acceptable excipients. The biologically active ingredient and the excipients may add up to 100 %.

In the case of a core which is an uncoated pellet, the coating with the ethanol resistance conferring coating layer has the functions of providing at first the desired release properties function to the pharmaceutical composition and secondly to provide resistance against the influence of ethanol.

The dosage form may be a tablet, a minitablet, a pellet, a granule, a sachet, a capsule, filled with coated pellets or with powder or with granules, or a coated capsule.

Pellets or granules may be used as cores or in compressed tablets. As a rough estimation, pellets may have a size in the range of 50 to 1500, 250 to 1250 µm (average diameter), while coated tablets may have a size in the range of above 1000 µm up to 25 mm (diameter or length). As a rule one can say the smaller the size of the pellet cores are, the higher the pellet coating weight gain needed. This is due to the comparably higher surface area of pellets compared to tablets.

The term pellet-containing tablet or compressed tablet is well known to a skilled person. Such a tablet may have a size of around 5 to 25 mm for instance. Usually, defined pluralities of small active ingredient containing pellets are compressed therein together with binding excipients to give the well-known tablet form. After oral ingestion and contact with the body fluid the tablet form is disrupted and the pellets are set free. The compressed tablet combines the advantage of the single dose form for ingestion with the advantages of a multiple form, for instance the dosage accuracy. In tablets containing comparably low amounts of excipients, preferably talcum but also other excipients, may be used in contrast to pellets.

The term minitablet is well known to the skilled person. A minitablet is smaller than the traditional tablet and may have a size of around 1 to 4 mm. The minitablet is, like a pellet, a single dosage form to be used in multiple dosages. In comparison to pellets, which may be in the same size, minitablets usually have the advantage of having more regular surfaces which can be coated more accurately and more uniformly. Minitablets may be provided enclosed in capsules, such as gelatine capsules. Such capsules disrupt after oral ingestion and contact with the gastric or intestinal fluids and the minitablets are set free. Another application of minitablets is the individual fine adjustment of the active ingredient dosage. In this case the patient may ingest a defined number of minitablets directly which matches to the severeness of the disease to cure but also to his individual body weight. A minitablet is different from pellet-containing compressed tablet as discussed above.

The term sachet is well known to the skilled person. It refers to small sealed package which contains the active ingredient often in pellet containing liquid form or also in dry pellet or powder form. The sachet itself is only the package form and is not intended to be ingested. The content of the sachet may be dissolved in water or as an advantageous feature may be soaked or ingested directly without further liquid. The latter is an advantageous feature for the patient when the dosage form shall be ingested in a situation where no water is available. The sachet is an alternative dosage form to tablets, minitablets or capsules.

The term capsule is well known to the skilled person. A capsule is like the sachet a container for pellets containing liquids or also dry pellets or powders. However in contrast to the sachet the capsule consists of pharmaceutically acceptable excipients such as gelatine or hydroxypropylmethylcellulose (HPMC) and is intended to be ingested like a tablet. The capsules disrupts after oral ingestion and contact with the gastric or intestinal fluids and the contained multiple units are set free. Capsules for pharmaceutical purposes are commercially available in different standardized sizes.

### Coating layer

The core of the dosage form is coated with a coating layer comprising the polymer as disclosed and optionally excipients, preferably pharmaceutical or nutraceutical acceptable excipients.

The coating layer may comprise at least 2, at least 5, at least 10, at least 20, at least 30, at least 40, at least 50, at least 60, at least 70, at least 80, at least 90 or 100 % by weight of the polymer as disclosed and claimed herein. The coating layer may comprise 2 to 100, 5 to 98, 10 to 90, 12 to 80, 15 to 70, 18 to 60 or 20 to 50 % by weight of the polymer as disclosed herein.

The coating layer may comprise up to 10, up to 20, up to 30, up to 40, up to 50, up to 60, up to 70, up to 80, up to 90, up to 95, up to 98 % by weight of excipients, preferably pharmaceutical or nutraceutical excipients (pharmaceutical or nutraceutical acceptable excipients). The coating layer may comprise 0 to 98, 2 to 95, 10 to 90, 20 to 88, 30 to 85, 40 to 82 or 50 to 80 % by weight of excipients. The polymer and the optionally comprised pharmaceutical or nutraceutical excipients may add up to 100 %.

### Excipients

Excipients are well known to a skilled person and formulated along with the biologically active ingredient and/or with the polymer as disclosed and claimed herein. All excipients used must be toxicologically safe and be used in pharmaceuticals or nutraceuticals without risk for patients or consumers.

The dosage form may comprise excipients, preferably pharmaceutical or nutraceutical acceptable excipients, selected from the group of antioxidants, brighteners, binding agents, flavouring agents, flow aids, fragrances, glidants, penetration-promoting agents, pigments, plasticizers, excipient polymers (different from the polymers as disclosed herein, for instance celluloses) pore-forming agents or stabilizers or combinations thereof. The pharmaceutically or nutraceutically acceptable excipients may be comprised in the core and/or in the coating layer comprising the polymer as disclosed. A pharmaceutical or nutraceutical acceptable excipient is an excipient, which is allowed to be used for the application in the pharmaceutical or nutraceutical field.

The coating layer may comprise up to 98, up to 95, up to 90, up to 80, up to 70, up to 50, up to 60, up to 50, up to 40, up to 30, up to 20, up to 10 % by weight or not any (0 %) excipients at all, respectively pharmaceutical or nutraceutically acceptable excipients. Preferably, except for the inventive polymer, no further polymers are present in the coating layer.

### Plasticizers

Plasticizers achieve through physical interaction with a polymer a reduction in the glass transition temperature and promote film formation, depending on the added amount. Suitable substances usually have a molecular weight of between 100 and 20 000 and comprise one or more hydrophilic groups in the molecule, e.g. hydroxyl, ester or amino groups.

Examples of suitable plasticizers are alkyl citrates, glycerol esters, alkyl phthalates, alkyl sebacates, sucrose esters, sorbitan esters, diethyl sebacate, dibutyl sebacate, propylenglycol and polyethylene glycols 200 to 12 000. Preferred plasticizers are triethyl citrate (TEC), acetyl triethyl citrate (ATEC), diethyl sebacate and dibutyl sebacate (DBS). Mention should additionally be made of esters which are usually liquid at room temperature, such as citrates, phthalates, sebacates or castor oil. Esters of citric acid and sebacinic acid are preferably used.

Addition of the plasticizer to the formulation can be carried out in a known manner, directly, in aqueous solution or after thermal pre-treatment of the mixture. It is also possible to employ mixtures of plasticizers. However, since the polymer as disclosed herein shows a minimum film forming temperature (MFFT) of 35 °C or lower, it is possible to apply the polymer coating, for instance from an aqueous polymer dispersion, without the addition of a plasticizer. Thus, the coating layer may comprise up to 25, up to 20, up to 15, up to 10, up to 5, but preferably less than 5% by weight calculated on the polymer of a plasticizer or any (0 %) plasticizer at all.

### Fillers

Standard fillers are usually added to the inventive formulation during processing to coating and binding agents. The quantities introduced and the use of standard fillers in pharmaceutical coatings or overlayers is familiar to those skilled in the art. Examples of standard fillers are release agents, pigments, stabilizers, antioxidants, pore-forming agents, penetration-promoting agents, brighteners, fragrances or flavouring agents. They are used as processing adjuvants and are intended to ensure a reliable and reproducible preparation process as well as good long-term storage stability, or they achieve additional advantageous properties in the pharmaceutical form. They are added to the polymer formulations before processing and can influence the permeability of the coatings. This property can be used if necessary as an additional control parameter.

### Glidants (Release Agents):

Glidants or release agents usually have lipophilic properties and are usually added to spray suspensions. They prevent agglomeration of cores during film formation. Suitable glidants are talc, Mg- or Ca-stearate, ground silica, kaolin or nonionic emulsifiers with an HLB value of between 2 and 8. Standard proportions for use of release agents in the inventive coating and binding agents range between 0.5 and 100 % by weight relative to polymer.

In a particularly advantageous embodiment, the glidant or release agent is added in concentrated form as the outer layer. Application takes place in the form of powder or by spraying from aqueous suspension with 5 to 30% (weight/weight (w/w)) solid content. The necessary concentration is lower than for incorporation into the polymer layer and amounts to 0.1 to 2% by weight relative to the weight of the pharmaceutical form.

The coating layer of the dosage form may for instance comprise 20 to 80, preferably 30 to 70 % by weight of the inventive polymer as disclosed and 20 to 80, 30 to 70 % by weight of talc. The inventive polymer and talc may add up to 100 % by weight.

### Pigments:

Only rarely is the pigment added in soluble form. As a rule, aluminum oxide or iron oxide pigments are used in dispersed form. Titanium dioxide is used as a whitening pigment. Standard proportions for use of pigments in the inventive coating and binding agents range between 10 to 200, 20 to 200 % by weight relative to the polymer. Because of the high pigment-binding capacity of the polymer, proportions up to 200 % by weight calculated on the polymer can be easily processed.

In a particularly advantageous embodiment, the pigment is used directly in concentrated form as an outer layer. Application takes place in the form of powder or by spraying from aqueous suspension with 5 to 35% (w/w) solid content. The necessary concentration is lower than for incorporation into the polymeric coating layer and amounts from about 0.1 to 2% by weight relative to the weight of the pharmaceutical form.

### Process for preparing the dosage form

A suitable process for preparing the dosage form as disclosed herein may be by forming a core comprising the active ingredient by direct compression, compression of dry, wet or sintered granules, by extrusion and subsequent rounding off, by wet or dry granulation, by direct pelleting or by binding powders onto active ingredient-free beads or neutral cores or active ingredient-containing particles or pellets and by applying the coating layer in the form of aqueous dispersions or organic solutions in spray processes or by fluidized bed spray granulation. The water content of aqueous dispersions comprising the polymer as disclosed and optional excipients may be in the range of 50 to 95, 60 to 85 or 65 to 80 % by weight. The polymer content of an aqueous dispersion may be in the range of 5 to 50, 15 to 40 or 20 to 35 % by weight.

### Use

Disclosed is also a polymer for use as, the use of a polymer or a method of the use of a polymer, as comprised in the dosage form disclosed herein, wherein the polymer is polymerized from a monomer mixture comprising the monomers
(a) 70 to 95% by weight of a C₁ to C₁₂ alkylester of acrylic acid or of methacrylic acid, preferably 2-Ethylhexyl methacrylate (EHMA) and Ethyl methacrylate (EMA) or 2-Ethylhexyl methacrylate (EHMA) and methyl methacrylate (MMA), mist preferred Ethylhexyl methacrylate (EHMA) and Ethyl methacrylate (EMA),
(b) 2.5 to 25, preferably 2.5 to 15 % by weight of a C₂ to C₆ hydroxy-alkylester of acrylic acid or methacrylic acid , preferably 2-Hydroxyethyl methacrylate,
(c) 2.5 to 15% by weight of a C₂ to C₈ alkyl ester of acrylic acid or of methacrylic acid with a quaternary cationic group in the alkyl group, preferably Trimethylammonium-ethyl-methacrylate-chloride (TMAEMC), for preparing a dosage form as disclosed with a sustained release profile and resistance against the influence of ethanol. The monomers (a), (b) and (c) may optionally add up to 100%.

### Top Coats and Sub Coats

The dosage according to the invention may be further coated with a sub coat or a top coat or both. A sub coat may be located between the core and the coating layer, comprising the polymer as disclosed. A sub coat may have the function to separate substances of the core from substances of the controlling layer, which may be incompatible with each other. The sub coat has essentially no influence on the active ingredient release characteristics. A subcoat is preferably essentially water-soluble, for instance it may consist of substances like hydroxypropyl-methyl-cellulose (HPMC) as a film former. The average thickness of the sub coat layer is very thin, for example not more than 15 µm, preferably not more than 10 µm.

A top coat is also preferably water-soluble or essentially water-soluble. A top coat may have the function of colouring the pharmaceutical or nutraceutical form or protecting from environmental influences for instance from moisture during storage. The top coat may consist out of a binder, for instance a water-soluble polymer like a polysaccharide or HPMC, or a sugar compound like saccharose. The top coat may further contain pharmaceutically or nutraceutically acceptable excipients like pigments or glidants in high amounts. The topcoat has essentially no influence on the release characteristics.

### Examples

### Abbreviations

TGEH = 2-Ethylhexyl thioglycolat
EHMA = Ethylhexyl methacrylate
EMA = Ethyl methacrylate
HEMA = Hydroxyethyl methacrylate
TMAEMC = Trimethylammonium-ethyl-methacrylate-chloride
SDS = Sodium dodecyl sulfate
TEC = Triethyl citrate
NM = EUDRAGIT^{®} NM, a copolymer comprising polymerized units from 70 % by weight ethly acrylate and 30 % by weight methyl methacrylate
RL = EUDRAGIT^{®} RL, a copolymer comprising polymerized units of from 60% by weight of methyl methacrylate, 30% by weight of ethyl acrylate and 10% by weight of 2-trimethylammoniumethyl methacrylate chloride.
RS = EUDRAGIT^{®} RS, a copolymer comprising polymerized units from 65% by weight of methyl methacrylate, 30% by weight of ethyl acrylate and 5% by weight of 2-trimethylammoniumethyl methacrylate chloride.

### Measurement methods

The measurement of the percentage amount of active ingredient released can be carried out, for example, by on-line UV spectroscopy at a wavelength suitable for the respective active compound. HPLC determination is also possible. The methodology is familiar to a person skilled in the art.

The release of active ingredient can be determined according to USP, in particular USP 32-NF27, General Chapter <711>, *Dissolution,* Apparatus 2 (basket), Method <724> "Delayed Release (Enteric Coated) Articles-General, General Drug Release Standard", Method B (100 rpm, 37°C), type I basket, with the following modification: The pharmaceutical forms are tested at pH 1.2 for the first 2 hours using 0.1 N HCI medium or at pH 6.8 using a phosphate buffer (European Pharmacopoeia (EP)), which corresponds to an artificial intestinal medium.

The measurement in the ethanol containing aqueous pH 1.2 medium is carried out using 40 % ethanol (w/w) in the medium. If appropriate or required for a certain controlled release pharmaceutical composition, depending on the active ingredient included and the type and size of the release of form (small or large pellet or small or large tablet) instead of the basket method the paddle method may be used with 50, 100 or 150 rpm.

*In vitro* drug release of coated pellets from Example 2 was tested in triplicates using USP I (basket) apparatus. Measurement was carried out at 150 RPM in 900 mL dissolution vessels. Dissolution was tested in 0.1 N HCI (pH 1.2) with and without 40%(w/w) EtOH for 2 h. Subsequently, medium was fully replaced by pH 6.8 EP buffer and drug release was monitored for another 8 h. API concentration was quantified via UV/VIS spectroscopy. Results are presented as mean average ± standard deviation, relatively to the total drug concentration in the respective vessel after homogenization.

The MFFT was determined according to the Standard of the International Organisation for Standardization DIN ISO 2115 with the exception of point 6.1 in that the maximum difference of the most distant metering points was set to 50 °C.

Gel permeation chromatography (GPC) was used to determine the number- and weight-average molecular weights (Mₙ, M_{w}) and the polydispersity (D) of the inventive polymers in the examples according to DIN 55672-1. Equipment consisted of four PSS SDV columns (Mainz, Germany) plus pre-column of the same type, a column oven operating at 35 °C, an Agilent (Series 1100, Santa Clara, USA) pump plus RI-detector of the same series. A 0.02 M solution of 2-(Diethylamino)ethylamine (DEAEA) in Tetrahydrofuran (THF) was used as eluent at a flow rate of 1 mL/min. Samples were dissolved in the eluent at concentrations of 2 mg/mL. For each measurement 100 µL polymer solution was injected. The values for Mn and Mw were calculated based on calibration curves generated by Poly(methyl methacrylate) standards.

EUDRAGIT^{®} reference samples were measured using the eluent N,N-dimethylacetamide (DMAc). Method for EUDRAGIT^{®} RL/RS is described in more detail by Adler M. et al. (e-Polymers, ISSN (Online) 1618-7229, ISSN (Print) 2197-4586, DOI: https://doi.org/10.1515/epoly.2005.5.1.602). Method for EUDRAGIT^{®} NM is described in more detail by Adler M. et al. (e-Polymers, ISSN (Online) 1618-7229, ISSN (Print) 2197-4586, DOI: https://doi.org/10.1515/epoly.2004.4.1.608).

DSC measurement of the dry polymer substance was conducted according to DIN EN ISO 11357-2 with a heating rate of 20 °C/min. The midpoint glass transition temperature *T*_{mg} was determined by half step height method as described in section 10.1.2 of DIN EN ISO 11357-2.

### Example 1: Emulsion polymerization

The procedure is described exemplarily for **Polymer 1** (see Table 1). All other polymers were manufactured in the same manner. Setup consisted of a 1 L reaction vessel equipped with lid, agitator, condenser, nitrogen inlet and thermal sensor. Heating was carried out by a thermostat controlled water bath. A dosage pump with silicone tubes was used to dose monomer emulsion into the reaction mixture. In a first step, 534.0 g of water and 6.6 g of sodium dodecyl sulfate (SDS 15, 15.0%(w/w) aqueous solution) were dosed into the reactor, purged with nitrogen and the mixture was then heated to 80 °C. In parallel, in a separate flask, monomer emulsion was prepared by mixing 21.3 g of SDS 15, 0.8 g of chain transfer agent (2-Ethylhexylthioglycolat, TGEH), 188.7 g (67.4%(w/w)) EHMA, 63.3 g (22.6%(w/w)) EMA, 14.0 g (5%(w/w)) HEMA, and 14.0 g (5.0%(w/w)) TMAEMC with 76.0 g of water. Stable emulsion was formed by stirring for 20 min. As soon as reaction mixture reached target temperature (80 °C), 6.0 mL of APS initiator (ammonium persulfate, 10%(w/w) aqueous solution) were pipetted into the reactor, followed by feeding of previously prepared monomer-emulsion. Feeding was carried out stepwise using two different rates (10 min at 1.5 g/min, followed by 120 min at 3.0 mg/min). During dosing, reaction temperature was held constant between 80 and 82 °C. After complete monomer addition, reaction mixture was stirred for 30 min at 80 °C and then allowed to cool down to room temperature. In total 28.0 g SDS 15 solution were used (4.2 g SDS, 1.5%(w/w) based on polymer weight). Theoretic solid content of the resulting polymer dispersion is 30%(w/w). Dispersion was finally filtered through a 250 um gauze. Filtrate and polymer coagulate in the reactor were collected and dried for gravimetric analysis. Experimental solid content of the final dispersion was 29.1 %(w/w), coagulate was <0.1%.

Table 1 summarizes the compositions of polymers 1 -10 (according to the invention), polymers 11 and 12 (comparative, not according to the invention) and commercially available polymers EUDRAGIT^{®} RL, EUDRAGIT^{®} RS and EUDRAGIT^{®} NM (comparative, not according to the invention) with sustained release characteristics.

Abbreviations in table 1: (% = % by weight, Da = Dalton, M_{w} = weight-average molecular weight, *T*_{mg} = midpoint glass transition temperature, MFFT = minimum film forming temperature, D = Dispersity Index)

**Table 1: Co-monomer composition of polymers together with analytical characterization parameters**

| | Co-monomer composition | | | | | | | | GPC | |
|---|---|---|---|---|---|---|---|---|---|---|
| Compound | EHMA [%] | EMA [%] | HEMA [%] | TMAEMC [%] | MMA [%] | EA [%] | *T*_{mg} [°C] | MFFT [°C] | M_{w} [Da] | D |
| **Polymer 1** | 67.4 | 22.6 | 5 | 5 | | | 17 | 23 | 93 100 | 1.90 |
| **Polymer 2** | 65.5 | 22.0 | 7.5 | 5 | | | 16 | 24 | 91 100 | 1.92 |
| **Polymer 3** | 63.6 | 21.4 | 10 | 5 | | | 17 | 24.5 | 90000 | 1.94 |
| **Polymer 4** | 61.8 | 20.7 | 12.5 | 5 | | | 17 | 20 | 90200 | 1.90 |
| **Polymer 5** | 65.5 | 22.0 | 5 | 7.5 | | | 15 | 22.5 | 86500 | 1.96 |
| **Polymer 6** | 63.6 | 21.4 | 7.5 | 7.5 | | | 18 | 19 | 85 100 | 1.92 |
| **Polymer 7** | 63.6 | 21.4 | 5 | 10 | | | 12 | 11.5 | 87 300 | 1.87 |
| **Polymer 8** | 61.8 | 20.7 | 7.5 | 10 | | | 10 | 11 | | |
| **Polymer 9** | 61.8 | 20.7 | 5 | 12.5 | | | 13.5 | 10.5 | | |
| **Polymer 10** | 59.9 | 20.1 | 7.5 | 12.5 | | | 14 | 10 | | |
| **Polymer 11** | 63.6 | 21.4 | 15 | 0 | | | 29 | 25.5 | | |
| **Polymer 12** | 66.8 | 22.4 | 0 | 10.8 | | | | 10.5 | | |
| **EUDRAGIT^{®} RL** | | | | 10 | 60 | 30 | 70 | 40.0 | 28800 | 3.7 |
| **EUDRAGIT^{®} RS** | | | | 5 | 65 | 30 | 65 | 45.0 | 33 200 | 2.6 |
| **EUDRAGIT^{®}** NM | | | | | 30 | 70 | 9 | 5.0 | 623 000 | 2.83 |
| **Polymer 13** | 59.9 | 20.1 | 15 | 5 | | | 22 | 9.5 | 93 700 | 1.9 |

**Example 2: Film-coating of polymer dispersion onto cores with different biologically active ingredients**

Three different APIs were investigated: (1) Caffeine citrate pellets (Lee Pharma Limited, 40% drug content, particle size determined by sieve analysis: not more than 10% retains on sieve #14 ASTM and not more than 10% passes through sieve #25 ASTM, ca. 1 mm mean pellet diameter), (2) Metoplolol succinate pellets (Lee Pharma Limited, 40% drug content, Particle size determined by sieve analysis: not more than 10% retains on sieve #16 ASTM and not more than 10% passes through sieve #25 ASTM. ca. 1 mm mean pellet diameter) and (3) Theophylline granules (BASF, pure drug, Particle size determined by sieve analysis: not more than 10% retains on sieve #25 ASTM and not more than 10% passes through sieve #40 ASTM, ca. 0.5 mm mean granule diameter).

36 g talc (100%(w/w) compared to dry polymer mass) were suspended in 204 g water and homogenized using ultra turrax for 15 minutes. Subsequently, the prepared suspension was mixed with 120 g polymer dispersion from Example 1 (~30%(w/w) polymer solid content) and stirred for one hour. The amount of water for suspension of talc was calculated to result in a final spraying suspension of 20%(w/w) solid content. Coating experiments were carried out on a Hüttlin (Schopfheim, Germany) Mycrolab (H00263), equipped with an ISMATEC (Wertheim, Germany) MCP flexible-tube pump (silicone tube, internal diameter 2 mm) and an inlet-air dehumidifier. A 0.8 mm spray nozzle was used. Atomizing air-pressure and micro-climate air-pressure were set to 0.6 and 0.4 bar, respectively. Product-bed temperature was held constant at -30 °C, air-flow rate at -20 m³/h, spray rate between 10 and 15 g/min/kg. Process was stopped at desired polymer weight gain. Curing was performed in open trays for 24 h at 60 °C.

EUDRAGIT^{®} NM, EUDRAGIT^{®} RL and EUDRAGIT^{®} RS were used for comparison and film-coatings were prepared in the same manner. In case of EUDRAGIT^{®} RL/EUDRAGIT^{®} RS, however, a plasticizer is required to enable film formation at applied spraying conditions. Therefore, Triethyl citrate (TEC, 20% (w/w) compared to dry polymer mass) was added to the EUDRAGIT^{®} RL/EUDRAGIT^{®} RS-talc mixture and stirred for one hour prior to spraying process. Curing of all EUDRAGIT^{®} coated dosage forms was performed according to Evonik standard recommendations (Application Guidelines 12^{th} ed.) in open trays at 40 °C for 24 hours.

### Example 3: Dissolution testing in pure media and hydroalcoholic media

*In vitro* drug release of coated pellets from Example 2 was tested in triplicates using USP I (basket) apparatus. Measurement was carried out at 150 RPM in 900 mL dissolution vessels. Dissolution was tested in 0.1 N HCI (pH 1.2) with and without 40%(w/w) EtOH for 2 h. Subsequently, medium was fully replaced by pH 6.8 EP buffer (without ethanol) and drug release was monitored for another 8 h. API concentration was quantified via UV/VIS spectroscopy. Results are presented as mean average, relatively to the total drug concentration in the respective vessel after homogenization.

**Table 2: Dissolution tests with and without ethanol on Caffeine citrate pellets**

| **Polymer** | | **Polymer 1** | | **Polymer 2** | | **Polymer 3** | | **Polymer 4** | | **Polymer 5** | | **Polymer 6** | |
|---|---|---|---|---|---|---|---|---|---|---|---|---|---|
| Core | | Caffeine citrate pellets | | Caffeine citrate pellets | | Caffeine citrate pellets | | Caffeine citrate pellets | | Caffeine citrate pellets | | Caffeine citrate pellets | |
| Talcum [%(w/w) compared to dry polymer mass] | | 100 | | 100 | | 100 | | 100 | | 100 | | 100 | |
| TEC [%(w/w) compared to dry polymer mass] | | 0 | | 0 | | 0 | | 0 | | 0 | | 0 | |
| Polymer weight gain [%(w/w) compared to drug substrate mass] | | 15 | | 15 | | 15 | | 15 | | 15 | | 15 | |
| **Active release without /with 40% EtOH (w/w)** | | | | | | | | | | | | | |
| | 5 min (pH 1.2 without /with EtOH) | 0.06 | 0.10 | 0.09 | 0.09 | 0.12 | 0.10 | 0.29 | 0.35 | 0.10 | 0.15 | 0.17 | 0.16 |
| | 30 min (pH 1.2 without /with EtOH) | 0.12 | 0.27 | 0.22 | 0.36 | 0.40 | 0.67 | 3.63 | 4.63 | 0.26 | 0.53 | 0.44 | 0.72 |
| | 1 h (pH 1.2 without /with EtOH) | 0.18 | 0.44 | 0.32 | 0.75 | 0.83 | 1.87 | **25.05** | **26.39** | 0.50 | 1.04 | 1.16 | 2.02 |
| | 1.5 h (pH 1.2 without /with EtOH) | 0.24 | 0.61 | 0.47 | 1.34 | 2.13 | 4.95 | **49.39** | **54.50** | 1.08 | 1.90 | 3.30 | 5.31 |
| | 2 h (pH 1.2 without /with EtOH) | 0.32 | 0.84 | 0.79 | 2.40 | 5.43 | 11.88 | **65.59** | **72.38** | 2.45 | 3.56 | 7.83 | 11.94 |
| | 2.5 h (pH 6.8 without EtOH) | 0.53 | 0.71 | 1.59 | 3.36 | **10.46** | **19.24** | **76.91** | **82.21** | 4.92 | 5.60 | **13.95** | **19.56** |
| | 3 h (pH 6.8 without EtOH) | 0.83 | 0.93 | 2.97 | 4.94 | **16.62** | **27.50** | 83.76 | 89.15 | 8.01 | 8.46 | **20.21** | **27.85** |
| | 3.5 h (pH 6.8 without EtOH) | 1.29 | 1.17 | 5.10 | 6.87 | **23.00** | **35.43** | 88.66 | 93.44 | **11.60** | **11.71** | **26.48** | **35.69** |
| | 4 h (pH 6.8 without EtOH) | 1.96 | 1.50 | 7.79 | 9.10 | **29.15** | **42.54** | 92.18 | 95.96 | **15.40** | **15.17** | **32.40** | 42.69 |
| | 5 h (pH 6.8 without EtOH) | 3.94 | 2.45 | **14.00** | **14.15** | **39.93** | **54.10** | 96.24 | 98.24 | **23.20** | **22.30** | **42.72** | **54.35** |
| | 6 h (pH 6.8 without EtOH) | 6.80 | 3.90 | **20.60** | **19.53** | **48.50** | **63.01** | 98.03 | 99.08 | **30.72** | **29.31** | **51.04** | **63.52** |
| | 7 h (pH 6.8 without EtOH) | **10.21** | **5.80** | **26.86** | **24.92** | **55.34** | **70.06** | 98.90 | 99.35 | **37.24** | **35.90** | **57.58** | **70.74** |
| | 8 h (pH 6.8 without EtOH) | **13.95** | **8.06** | **32.69** | **30.17** | **61.11** | **75.72** | 99.26 | 99.54 | **42.88** | **41.87** | 63.03 | **76.51** |
| | 9 h (pH 6.8 without EtOH) | **17.96** | **10.58** | **38.20** | **35.12** | **66.10** | **80.30** | 99.38 | 99.60 | **48.20** | **47.14** | **68.42** | **81.01** |
| | 10 h (pH 6.8 without EtOH) | **21.99** | **13.28** | **43.10** | **39.71** | **70.55** | **84.14** | 99.41 | 99.58 | **52.93** | **51.85** | **73.30** | **84.72** |
| Arithmetic average (bold figures) | | -6.60 | | -1.98 | | 13.13 | | 4.64 | | -0.87 | | 10.75 | |
| **n** for calculation | | 4 | | 6 | | 10 | | 4 | | 8 | | 10 | |
| Ethanol resistance | | yes | | yes | | yes | | yes | | yes | | yes | |

**Table 3: Dissolution tests with and without ethanol on Caffeine citrate pellets**

| **Polymer No.** | | **Polymer 7** | | **Polymer 8** | | **Polymer 9** | | **Polymer 10** | | **NM** | | **RS/RL (9 : 1)** | |
|---|---|---|---|---|---|---|---|---|---|---|---|---|---|
| Core | | Caffeine citrate pellets | | Caffeine citrate pellets | | Caffeine citrate pellets | | Caffeine citrate pellets | | Caffeine citrate pellets | | Caffeine citrate pellets | |
| Talcum [%(w/w) compared to dry polymer mass] | | 100 | | 100 | | 100 | | 100 | | 100 | | 50 | |
| TEC [%(w/w) compared to dry polymer mass] | | 0 | | 0 | | 0 | | 0 | | 0 | | 20 | |
| Polymer weight gain [%(w/w) compared to drug substrate mass] | | 15 | | 15 | | 15 | | 15 | | 6 | | 15 | |
| **Active release without /with 40% EtOH (w/w)** | | | | | | | | | | | | | |
| | 5 min (pH 1.2 without /with EtOH) | 0 | 0 | 0 | 0 | 0.21 | 0.16 | 0.39 | 0.32 | 0.01 | 1.62 | 0.49 | 99.57 |
| | 30 min (pH 1.2 without /with EtOH) | 0.15 | 0.12 | 1.89 | 1.96 | 1.78 | 1.57 | **10.27** | **6.65** | 0.10 | 37.99 | 1.23 | 99.88 |
| | 1 h (pH 1.2 without /with EtOH) | 1.09 | 1.31 | **13.37** | **10.64** | 9.00 | 5.91 | **35.77** | **28.69** | 0.25 | 73.03 | 1.80 | 99.88 |
| | 1.5 h (pH 1.2 without /with EtOH) | 3.44 | 3.59 | **31.01** | **30.03** | **20.94** | **14.78** | **56.97** | **55.90** | 0.66 | 87.80 | 2.75 | 99.76 |
| | 2 h (pH 1.2 without /with EtOH) | 7.64 | 7.50 | **46.83** | **51.51** | **33.51** | **27.35** | **70.65** | **73.37** | 2.68 | 94.71 | **12.26** | **99.97** |
| | 2.5 h (pH 6.8 without EtOH) | **12.82** | **12.11** | **58.73** | **67.28** | **44.55** | **40.08** | 81.04 | 85.61 | 6.42 | 96.49 | **41.64** | **99.99** |
| | 3 h (pH 6.8 without EtOH) | **18.43** | **17.45** | **66.92** | **78.28** | **52.39** | **51.31** | 87.36 | 92.65 | 9.93 | 97.52 | **70.44** | **99.99** |
| | 3.5 h (pH 6.8 without EtOH) | **24.09** | **22.87** | **73.51** | **85.77** | **58.78** | **60.50** | 91.62 | 96.08 | **13.12** | **98.22** | **79.95** | **99.99** |
| | 4 h (pH 6.8 without EtOH) | **29.52** | **28.07** | **78.82** | **90.61** | **64.19** | **67.67** | 94.33 | 97.67 | **16.01** | **98.71** | 85.87 | 99.99 |
| | 5 h (pH 6.8 without EtOH) | **39.38** | **37.84** | 86.63 | 95.91 | **72.96** | **78.08** | 97.15 | 98.80 | **21.69** | **99.26** | 89.42 | 99.96 |
| | 6 h (pH 6.8 without EtOH) | **47.59** | **46.30** | 91.60 | 97.97 | **79.78** | **84.98** | 98.26 | 99.10 | **26.86** | **99.52** | 91.72 | 99.90 |
| | 7 h (pH 6.8 without EtOH) | **54.43** | **53.53** | 94.64 | 98.83 | 85.10 | 89.59 | 98.73 | 99.21 | **31.12** | **99.66** | 93.17 | 99.96 |
| | 8 h (pH 6.8 without EtOH) | **60.37** | **59.73** | 96.49 | 99.18 | 89.18 | 92.65 | 98.96 | 99.26 | **34.73** | **99.70** | 94.96 | 99.96 |
| | 9 h (pH 6.8 without EtOH) | **65.39** | **64.95** | 97.54 | 99.29 | 92.27 | 94.79 | 99.06 | 99.32 | **37.86** | **99.76** | 96.01 | 99.90 |
| | 10 h (pH 6.8 without EtOH) | **70.08** | **69.69** | 98.30 | 99.52 | 94.48 | 96.17 | 99.12 | 99.35 | **40.71** | **99.76** | 96.59 | 99.91 |
| Arithmetic average (bold figures) | | -0.95 | | 6.42 | | -0.30 | | -2.26 | | 71.56 | | 48.91 | |
| **n** for calculation | | 10 | | 7 | | 8 | | 4 | | 8 | | 4 | |
| Ethanol resistance | | yes | | yes | | yes | | yes | | no | | no | |

**Table 4: Dissolution tests with and without ethanol on Metoprolol succinate pellets**

| **Polymer No.** | | **Polymer 3** | | **Polymer 6** | | **Polymer 11** | | **Polymer 12** | | **NM** | | **RS/RL (9 : 1)** | |
|---|---|---|---|---|---|---|---|---|---|---|---|---|---|
| Core | | Metoplolol scc. pellets | | Metoplolol scc. pellets | | Metoplolol scc. pellets | | Metoplolol scc. pellets | | Metoplolol scc. pellets | | Metoplolol scc. pellets | |
| Talcum [%(w/w) compared to dry polymer mass] | | 100 | | 100 | | 100 | | 100 | | 100 | | 50 | |
| TEC [%(w/w) compared to dry polymer mass] | | 0 | | 0 | | 10 | | 0 | | 0 | | 20 | |
| Polymer weight gain [%(w/w) compared to drug substrate mass] | | 15 | | 15 | | 10 | | 10 | | 6 | | 15 | |
| **Active release without /with 40% EtOH (w/w)** | | | | | | | | | | | | | |
| | 5 min (pH 1.2 without /with EtOH) | 0 | 0 | 0 | 0 | 0.06 | 0.17 | 0 | 0 | 0.03 | 0.44 | 2.04 | 71.06 |
| | 30 min (pH 1.2 without /with EtOH) | 0.28 | 0.97 | 2.56 | 1.66 | 0.09 | 2.69 | 2.08 | 0.79 | 1.03 | 7.51 | 4.33 | 102.18 |
| | 1 h (pH 1.2 without /with EtOH) | 2.75 | 4.07 | 14.72 | 8.51 | 0.12 | 46.57 | 11.66 | 3.04 | 4.18 | 23.76 | 5.29 | 100.84 |
| | 1.5 h (pH 1.2 without /with EtOH) | 10.24 | 11.55 | 32.72 | 19.44 | 0.16 | 77.51 | 27.09 | 7.01 | 8.57 | 38.88 | 5.90 | 100.30 |
| | 2 h (pH 1.2 without /with EtOH) | 21.85 | 22.71 | 49.48 | 32.03 | 0.23 | 88.78 | 43.26 | 12.76 | 13.92 | 67.88 | 11.04 | 99.71 |
| | 2.5 h (pH 6.8 without EtOH) | 32.73 | 29.74 | 62.17 | 39.25 | 0.34 | 91.70 | 55.26 | 13.07 | 19.28 | 78.26 | 25.56 | 99.79 |
| | 3 h (pH 6.8 without EtOH) | **42.49** | **36.60** | **70.39** | **46.92** | 0.46 | 93.17 | **63.45** | **16.42** | **24.52** | **81.25** | **36.87** | **99.81** |
| | 3.5 h (pH 6.8 without EtOH) | **51.20** | **43.26** | **76.62** | **54.11** | 0.68 | 94.31 | **69.92** | **20.42** | **27.77** | **82.88** | **41.59** | **99.81** |
| | 4 h (pH 6.8 without EtOH) | **58.80** | **49.50** | 81.35 | 60.47 | 1.23 | 95.16 | **75.13** | **24.74** | **40.64** | **87.97** | **45.87** | **99.82** |
| | 5 h (pH 6.8 without EtOH) | **70.67** | **60.19** | 87.89 | 70.79 | 5.15 | 96.40 | 82.75 | 33.84 | **51.72** | **91.09** | **49.54** | **99.82** |
| | 6 h (pH 6.8 without EtOH) | **78.93** | **68.70** | 91.96 | 78.37 | **15.18** | **97.27** | 87.88 | 42.74 | **60.61** | **93.47** | **52.83** | **99.85** |
| | 7 h (pH 6.8 without EtOH) | 84.55 | 75.16 | 94.48 | 83.95 | **33.79** | **97.80** | 91.37 | 50.84 | **67.68** | **95.10** | **55.63** | **99.84** |
| | 8 h (pH 6.8 without EtOH) | 88.47 | 80.20 | 96.08 | 88.09 | **53.48** | **98.20** | 93.87 | 57.94 | **73.39** | **96.16** | **60.27** | **99.82** |
| | 9 h (pH 6.8 without EtOH) | 91.13 | 84.03 | 97.09 | 91.10 | **67.35** | **98.45** | 95.60 | 63.86 | **77.98** | **96.94** | **63.67** | **99.85** |
| | 10 h (pH 6.8 without EtOH) | 93.05 | 86.91 | 97.72 | 93.32 | **75.51** | **98.64** | 96.74 | 69.07 | 81.77 | 97.45 | **66.33** | **99.88** |
| Arithmetic average (bold figures) | | -5.58 | | -17.64 | | 49.01 | | -35.47 | | 41.35 | | 53.53 | |
| n for calculation | | 8 | | 6 | | 5 | | 7 | | 10 | | 11 | |
| Ethanol resistance | | yes | | yes | | no | | no | | no | | no | |

**Table 5: Dissolution tests with and without ethanol on Theophylline granules and Caffeine citrate pellets**

| **Polymer No.** | | **Polymer 8** | | **NM** | | **RS/RL (3 : 7)** | | **Polymer 13** | |
|---|---|---|---|---|---|---|---|---|---|
| Core | | Theophylline granules | | Theophylline granules | | Theophylline granules | | Caffeine citrate pellets | |
| Talcum [%(w/w) compared to dry polymer mass] | | 100 | | 100 | | 100 | | 100 | |
| TEC [%(w/w) compared to dry polymer mass] | | 0 | | 0 | | 20 | | 0 | |
| Polymer weight gain [%(w/w) compared to drug substrate mass] | | 6 | | 6 | | 10 | | 15 | |
| **Active release without /with 40% EtOH (w/w)** | | | | | | | | | |
| | 5 min (pH 1.2 without /with EtOH) | 1.36 | 1.75 | 1.21 | 4.23 | 2.57 | 87.71 | 0.22 | 0.35 |
| | 30 min (pH 1.2 without /with EtOH) | 6.95 | 7.53 | 5.09 | 22.54 | **14.11** | **102.32** | 2.52 | 3.70 |
| | 1 h (pH 1.2 without /with EtOH) | **13.18** | **13.22** | 9.73 | 38.60 | **28.60** | **101.02** | **10.47** | **14.74** |
| | 1.5 h (pH 1.2 without /with EtOH) | **18.94** | **18.35** | **14.13** | **51.15** | **43.99** | **100.13** | **22.45** | **31.59** |
| | 2 h (pH 1.2 without /with EtOH) | **24.36** | **23.22** | **18.37** | **60.93** | **58.67** | **99.74** | **35.36** | **49.42** |
| | 2.5 h (pH 6.8 without EtOH) | **28.45** | **26.50** | **21.70** | **65.17** | **69.13** | **99.96** | **54.06** | **61.10** |
| | 3 h (pH 6.8 without EtOH) | **32.13** | **29.46** | **25.20** | **67.88** | **77.78** | **99.96** | **60.49** | **70.66** |
| | 3.5 h (pH 6.8 without EtOH) | **35.59** | **32.32** | **28.67** | **70.31** | 84.67 | 99.96 | | |
| | 4 h (pH 6.8 without EtOH) | **38.81** | **35.09** | **32.08** | **72.50** | 89.86 | 99.97 | **72.07** | **83.87** |
| | 5 h (pH 6.8 without EtOH) | **44.67** | **40.33** | **38.73** | **76.37** | 96.04 | 99.96 | 80.16 | 91.23 |
| | 6 h (pH 6.8 without EtOH) | **49.83** | **45.17** | **45.10** | **79.73** | 98.51 | 99.97 | 86.65 | 95.22 |
| | 7 h (pH 6.8 without EtOH) | **54.38** | **49.63** | **51.11** | **82.43** | 99.36 | 99.96 | 91.26 | 97.27 |
| | 8 h (pH 6.8 without EtOH) | **58.38** | **53.72** | **56.72** | **84.66** | 99.61 | 99.96 | 94.32 | 98.30 |
| | 9 h (pH 6.8 without EtOH) | **61.69** | **57.47** | **61.89** | **86.61** | 99,33 | 99.97 | 96.31 | 98.83 |
| | 10 h (pH 6.8 without EtOH) | **64.65** | **60.84** | **66.67** | **88.15** | 99.15 | 99.97 | 97.57 | 99.17 |
| Arithmetic average (bold figures) | | -3.06 | | 35.46 | | 51.81 | | 9.41 | |
| **n** for calculation | | 13 | | 12 | | 6 | | 6 | |
| Ethanol resistance | | yes | | no | | no | | yes | |

## Claims

1. Polymer, polymerized from a monomer mixture comprising
(a) 70 to 95% by weight of 2-Ethylhexyl methacrylate (EHMA) and Ethyl methacrylate (EMA) or 2-Ethylhexyl methacrylate (EHMA) and methyl methacrylate (MMA)
(b) 2.5 to 25% by weight of one or more C₂ to C₆ hydroxy-alkylesters of acrylic acid or methacrylic acid,
(c) 2.5 to 15% by weight of one or more C₂ to C₈ alkyl esters of acrylic acid or of methacrylic acid with a quaternary cationic group in the alkyl group.

2. Polymer as claimed in Claim 1, comprising
(a) 70 to 95% by weight of 2-Ethylhexyl methacrylate (EHMA) and Ethyl methacrylate (EMA) or 2-Ethylhexyl methacrylate (EHMA) and methyl methacrylate (MMA)
(b) 2.5 to 15% by weight of one or more C₂ to C₆ hydroxy-alkylesters of acrylic acid or methacrylic acid,
(c) 2.5 to 15% by weight of one or more C₂ to C₈ alkyl esters of acrylic acid or of methacrylic acid with a quaternary cationic group in the alkyl group.

3. Polymer as claimed in Claim 1 or 2, wherein 2-Ethylhexyl methacrylate (EHMA) and Ethyl methacrylate (EMA) or 2-Ethylhexyl methacrylate (EHMA) and methyl methacrylate (MMA) are comprised in a ratio by weight from 5 : 1 to 1 : 1.

4. Polymer as claimed any of Claims 1 to 3, wherein the C₂ to C₆ hydroxy-alkylesters of acrylic acid or of methacrylic acid (b) are selected from 2-Hydroxyethyl methacrylate (HEMA), 2-Hydroxypropyl methacrylate, 3-Hydroxypropyl methacrylate, 2,3-Dihydroxypropyl methacrylate, 2-Hydroxyethyl acrylate, 2-Hydroxypropyl acrylate, 3-Hydroxypropyl acrylate and 2,3-Dihydroxypropyl acrylate or any mixture thereof.

5. Polymer as claimed in any of Claims 1 to 4, wherein the C₂ to C₈ alkyl ester of acrylic acid or of methacrylic acid with a quaternary cationic group in the alkyl group (c) is 2-Trimethylammonium-ethyl-methacrylate-Chloride (TMAEMC) or 2-Trimethylammonium-propyl-methacrylate-Chloride (TMAPMC) or both or, most preferred 2-Trimethylammonium-ethyl-methacrylate-Chloride (TMAEMC).

6. Polymer as claimed in any of Claims 1 to 5, wherein the monomer mixture comprises less than 10 % by weight of a monomer (d) which is acrylic acid or methacrylic acid or both.

7. Polymer as claimed in any of Claims 1 to 6, polymerized from a monomer mixture comprising (a1), (a2), (b) and (c) and optionally (d) with
(a1) 40 to 80 % by weight 2-Ethylhexyl methacrylate,
(a2) 15 to 30 % by weight Ethyl methacrylate,
(b) 2.5 to 15 % by weight 2-Hydroxyethyl methacrylate,
(c) 2.5 to 15 % by weight 2-Trimethylammonium-ethyl-methacrylate-chloride and optionally
(d) 0 up to less than 10 % by weight acrylic acid or methacrylic acid,
wherein (a1), (a2), (b) and (c) and optionally (d) add up to 100 %.

8. Polymer as claimed in any of Claims 1 to 7, wherein the minimum film forming temperature (MFFT) is 35 °C or lower and wherein the minimum film forming temperature (MFFT) is determined according to DIN ISO 2115.

9. Polymer as claimed in any of Claims 1 to 8 wherein the midpoint glass transition temperature (*T*_{mg}) is in the range from 0 to 50 °C and wherein the midpoint glass transition temperature (Tmg) of the polymer is determined by DSC measurement according to DIN EN ISO 11357-2.

10. Polymer as claimed in any of Claims 1 to 9, wherein the weight average molecular weight Mw is from 10.000 to 200.000 Dalton and wherein the weight average molecular weight is determined by Gel Permeation Chromatography according to DIN 55672-1.

11. Dosage form, comprising a core, comprising a biologically active ingredient, and a coating layer onto the core, wherein the coating layer is comprising a polymer according to one or more of Claims 1 to 10 or a polymer, polymerized from a monomer mixture comprising the monomers
(a) 70 to 95% by weight of a C₁ to C₁₂ alkylester of acrylic acid or of methacrylic acid,
(b) 2.5 to 25% by weight of a C₂ to C₆ hydroxy-alkylester of acrylic acid or methacrylic acid
(c) 2.5 to 15% by weight of a C₂ to C₈ alkyl ester of acrylic acid or of methacrylic acid with a quaternary cationic group in the alkyl group.

12. Dosage form, according to Claim 11, wherein the coating layer comprises a polymer according to one or more of Claims 1 to 10.

13. Dosage form as claimed in Claim 11 or 12, wherein the coating layer does not contain a plasticizer or less than 5 % by weight calculated on the weight of the polymer.

14. Use of the dosage form according to one or more of Claims 11 to 13, as an orally delivered medicament with resistance against the influence of ethanol.

15. Process for preparing a polymer as claimed in any of Claims 1 to 10 from a monomer mixture in the presence of a polymerisation initiator and optionally a Chain-transfer agent by bulk polymerisation, suspension polymerisation or emulsion polymerisation.

16. The use of a polymer, polymerized from a monomer mixture comprising the monomers
(a) 70 to 95% by weight of a C₁ to C₁₂ alkylester of acrylic acid or of methacrylic acid,
(b) 2.5 to 25% by weight of a C₂ to C₆ hydroxy-alkylester of acrylic acid or methacrylic acid,
(c) 2.5 to 15% by weight of a C₂ to C₈ alkyl ester of acrylic acid or of methacrylic acid with a quaternary cationic group in the alkyl group.
for preparing a dosage form as claimed in one or more of Claims 10 to 13 with a sustained release profile and resistance against the influence of ethanol.

17. Use as claimed in Claim 16, comprising a polymer, polymerized from a monomer mixture comprising the monomers
(a) 70 to 95% by weight of a C₁ to C₁₂ alkylester of acrylic acid or of methacrylic acid,
(b) 2.5 to 15% by weight of one or more C₂ to C₆ hydroxy-alkylesters of acrylic acid or methacrylic acid, and
(c) 2.5 to 15% by weight of one or more C₂ to C₈ alkyl esters of acrylic acid or of methacrylic acid with a quaternary cationic group in the alkyl group.

## Patentansprüche

1. Polymer, polymerisiert aus einer Monomerenmischung, umfassend
(a) 70 bis 95 Gew.-% 2-Ethylhexylmethacrylat (EHMA) und Ethylmethacrylat (EMA) oder 2-Ethylhexylmethacrylat (EHMA) und Methylmethacrylat (MMA),
(b) 2,5 bis 25 Gew.-% eines oder mehrerer C₂- bis C₆-Hydroxylalkylester der Acrylsäure oder der Methacrylsäure,
(c) 2,5 bis 15 Gew.-% eines oder mehrerer C₂- bis C₈-Alkylester der Acrylsäure oder der Methacrylsäure mit einer quaternären kationischen Gruppe in der Alkylgruppe.

2. Polymer nach Anspruch 1, umfassend
(a) 70 bis 95 Gew.-% 2-Ethylhexylmethacrylat (EHMA) und Ethylmethacrylat (EMA) oder 2-Ethylhexylmethacrylat (EHMA) und Methylmethacrylat (MMA),
(b) 2,5 bis 15 Gew.-% eines oder mehrerer C₂- bis C₆-Hydroxylalkylester der Acrylsäure oder der Methacrylsäure,
(c) 2,5 bis 15 Gew.-% eines oder mehrerer C₂- bis C₈-Alkylester der Acrylsäure oder der Methacrylsäure mit einer quaternären kationischen Gruppe in der Alkylgruppe.

3. Polymer nach Anspruch 1 oder 2, wobei 2-Ethylhexylmethacrylat (EHMA) und Ethylmethacrylat (EMA) oder 2-Ethylhexylmethacrylat (EHMA) und Methylmethacrylat in einem Gewichtsverhältnis von 5:1 bis 1:1 enthalten sind.

4. Polymer nach einem der Ansprüche 1 bis 3, wobei die C₂- bis C₆-Hydroxylalkylester der Acrylsäure oder der Methacrylsäure (b) aus 2-Hydroxyethylmethacrylat (HEMA), 2-Hydroxypropylmethacrylat, 3-Hydroxypropylmethacrylat, 2,3-Dihydroxypropylmethacrylat, 2-Hydroxyethylacrylat, 2-Hydroxypropylacrylat, 3-Hydroxypropylacrylat und 2,3-Dihydroxypropylacrylat oder einer beliebigen Mischung davon ausgewählt sind.

5. Polymer nach einem der Ansprüche 1 bis 4, wobei es sich bei dem C₂- bis C₈-Alkylester der Acrylsäure oder der Methacrylsäure mit einer quaternären kationischen Gruppe in der Alkylgruppe (c) um 2-Trimethylammoniumethylmethacrylatchlorid (TMAEMC) oder 2-Trimethylammoniumpropylmethacrylatchlorid (TMAPMC) oder beide oder ganz besonders bevorzugt 2-Trimethylammoniumethylmethacrylatchlorid (TMAEMC) handelt.

6. Polymer nach einem der Ansprüche 1 bis 5, wobei die Monomerenmischung weniger als 10 Gew.-% eines Monomers (d), bei dem es sich um Acrylsäure oder Methacrylsäure oder beide handelt, umfasst.

7. Polymer nach einem der Ansprüche 1 bis 6, polymerisiert aus einer Monomerenmischung umfassend (a1), (a2), (b) und (c) und gegebenenfalls (d), mit
(a1) 40 bis 80 Gew.-% 2-Ethylhexylmethacrylat,
(a2) 15 bis 30 Gew.-% Ethylmethacrylat,
(b) 2,5 bis 15 Gew.-% 2-Hydroxyethylmethacrylat,
(c) 2,5 bis 15 Gew.-% 2-Trimethylammoniummethylmethacrylatchlorid und gegebenenfalls
(d) 0 bis weniger als 10 Gew.-% Acrylsäure oder Methacrylsäure,
wobei sich (a1), (a2), (b) und (c) und gegebenenfalls (d) zu 100 Gew.-% summieren.

8. Polymer nach einem der Ansprüche 1 bis 7, wobei die Mindestfilmbildungstemperatur (MFFT) 35 °C oder weniger beträgt und wobei die Mindestfilmbildungstemperatur (MFFT) gemäß DIN ISO 2115 bestimmt wird.

9. Polymer nach einem der Ansprüche 1 bis 8, wobei die Midpoint-Glasübergangstemperatur (*T*_{mg}) im Bereich von 0 bis 50 °C liegt und wobei die Midpoint-Glasübergangstemperatur (Tmg) des Polymers durch DSC-Messung gemäß DIN EN ISO 11357-2 bestimmt wird.

10. Polymer nach einem der Ansprüche 1 bis 9, wobei das gewichtsmittleren Molekulargewicht Mw 10.000 bis 200.000 Dalton beträgt und wobei das gewichtsmittlere Molekulargewicht durch Gelpermeationschromatographie gemäß DIN 55672-1 bestimmt wird.

11. Dosierungsform, umfassend einen Kern, der einen biologischen Wirkstoff umfasst, und eine Überzugsschicht auf dem Kern, wobei die Überzugsschicht aus einem Polymer nach einem oder mehreren der Ansprüche 1 bis 10 oder einem aus einer Monomerenmischung, die die Monomere
(a) 70 bis 95 Gew.-% eines C₁- bis C₁₂-Alkylesters der Acrylsäure oder der Methacrylsäure,
(b) 2,5 bis 25 Gew.-% eines C₂- bis C₆-Hydroxylalkylesters der Acrylsäure oder der Methacrylsäure,
(c) 2,5 bis 15 Gew.-% eines C₂- bis C₈-Alkylesters der Acrylsäure oder der Methacrylsäure mit einer quaternären kationischen Gruppe in der Alkylgruppe umfasst, polymerisierten Polymer besteht.

12. Dosierungsform nach Anspruch 11, wobei die Überzugsschicht ein Polymer nach einem oder mehreren der Ansprüche 1 bis 10 umfasst.

13. Dosierungsform nach Anspruch 11 oder 12, wobei die Überzugsschicht keinen Weichmacher oder weniger als 5 Gew.-%, bezogen auf das Gewicht des Polymers, enthält.

14. Verwendung der Dosierungsform nach einem oder mehreren der Ansprüche 11 bis 13 als oral zugeführtes Medikament mit Beständigkeit gegenüber dem Einfluss von Ethanol.

15. Verfahren zur Herstellung eines Polymers nach einem der Ansprüche 1 bis 10 aus einer Monomerenmischung in Gegenwart eines Polymerisationsinitiators und gegebenenfalls eines Ketten-übertragungsmittels durch Massenpolymerisation, Suspensionspolymerisationen oder Emulsionspolymerisation.

16. Verwendung eines Polymers, polymerisiert aus einer Monomerenmischung, die die Monomere
(a) 70 bis 95 Gew.-% eines C₁- bis C₁₂-Alkylesters der Acrylsäure oder der Methacrylsäure,
(b) 2,5 bis 25 Gew.-% eines C₂- bis C₆-Hydroxylalkylesters der Acrylsäure oder der Methacrylsäure,
(c) 2,5 bis 15 Gew.-% eines C₂- bis C₈-Alkylesters der Acrylsäure oder der Methacrylsäure mit einer quaternären kationischen Gruppe in der Alkylgruppe umfasst, zur Herstellung einer Dosierungsform nach einem oder mehreren der Ansprüche 10 bis 13 mit einem Retard Profil und Beständigkeit gegenüber dem Einfluss von Ethanol.

17. Verwendung nach Anspruch 16, umfassend ein aus einer Monomerenmischung, die die Monomere
(a) 70 bis 95 Gew.-% eines C₁- bis C₁₂-Alkylesters der Acrylsäure oder der Methacrylsäure,
(b) 2,5 bis 15 Gew.-% eines C₂- bis C₆-Hydroxylalkylesters der Acrylsäure oder der Methacrylsäure,
(c) 2,5 bis 15 Gew.-% eines oder mehrerer C₂- bis C₈-Alkylester der Acrylsäure oder der Methacrylsäure mit einer quaternären kationischen Gruppe in der Alkylgruppe
umfasst, polymerisiertes Polymer.

## Revendications

1. Polymère, polymérisé à partir d'un mélange de monomères comprenant
(a) 70 à 95 % en poids de méthacrylate de 2-éthylhexyle (EHMA) et de méthacrylate d'éthyle (EMA) ou de méthacrylate de 2-éthylhexyle (EHMA) et de méthacrylate de méthyle (MMA)
(b) 2,5 à 25 % en poids d'un ou plusieurs esters d'hydroxyalkyle en C₂ à C₆ d'acide acrylique ou d'acide méthacrylique,
(c) 2,5 à 15 % en poids d'un ou plusieurs esters d'alkyle en C₂ à C₈ d'acide acrylique ou d'acide méthacrylique comportant un groupe cationique quaternaire dans le groupe alkyle.

2. Polymère selon la revendication 1, comprenant
(a) 70 à 95 % en poids de méthacrylate de 2-éthylhexyle (EHMA) et de méthacrylate d'éthyle (EMA) ou de méthacrylate de 2-éthylhexyle (EHMA) et de méthacrylate de méthyle (MMA)
(b) 2,5 à 15 % en poids d'un ou plusieurs esters d'hydroxyalkyle en C₂ à C₆ d'acide acrylique ou d'acide méthacrylique,
(c) 2,5 à 15 % en poids d'un ou plusieurs esters d'alkyle en C₂ à C₈ d'acide acrylique ou d'acide méthacrylique comportant un groupe cationique quaternaire dans le groupe alkyle.

3. Polymère selon la revendication 1 ou 2, le méthacrylate de 2-éthylhexyle (EHMA) et le méthacrylate d'éthyle (EMA) ou le méthacrylate de 2-éthylhexyle (EHMA) et le méthacrylate de méthyle (MMA) étant compris en un rapport en poids de 5:1 à 1:1.

4. Polymère selon l'une quelconque des revendications 1 à 3, les esters d'hydroxyalkyle en C₂ à C₆ d'acide acrylique ou d'acide méthacrylique (b) étant choisis parmi le méthacrylate de 2-hydroxyéthyle (HEMA), le méthacrylate de 2-hydroxypropyle, le méthacrylate de 3-hydroxypropyle, le méthacrylate de 2,3-dihydroxypropyle, l'acrylate de 2-hydroxyéthyle, l'acrylate de 2-hydroxypropyle, l'acrylate de 3-hydroxypropyle et l'acrylate de 2,3-dihydroxypropyle et un quelconque mélange correspondant.

5. Polymère selon l'une quelconque des revendications 1 à 4, l'ester d'alkyle en C₂ à C₈ d'acide acrylique ou d'acide méthacrylique comportant un groupe cationique quaternaire dans le groupe alkyle (c) étant le chlorure de méthacrylate de 2-triméthylammonium-éthyle (TMAEMC) ou le chlorure de méthacrylate de 2-triméthylammonium-propyle (TMAPMC) ou les deux ou, de la manière la plus préférée le chlorure de méthacrylate de 2-triméthylammonium-éthyle (TMAEMC).

6. Polymère selon l'une quelconque des revendications 1 à 5, le mélange de monomères comprenant moins de 10 % en poids d'un monomère (d) qui est l'acide acrylique ou l'acide méthacrylique ou les deux.

7. Polymère selon l'une quelconque des revendications 1 à 6, polymérisé à partir d'un mélange de monomères comprenant (a1), (a2), (b) et (c) et éventuellement (d) avec
(a1) 40 à 80 % en poids de méthacrylate de 2-éthylhexyle,
(a2) 15 à 30 % en poids de méthacrylate d'éthyle,
(b) 2,5 à 15 % en poids de méthacrylate de 2-hydroxyéthyle,
(c) 2,5 à 15 % en poids de chlorure de méthacrylate de 2-triméthylammonium-éthyle et éventuellement
(d) 0 jusqu'à moins de 10 % en poids d'acide acrylique ou d'acide méthacrylique,
(a1), (a2), (b) et (c) et éventuellement (d) totalisant jusqu'à 100 %.

8. Polymère selon l'une quelconque des revendications 1 à 7, la température minimale de formation de film (MFFT) étant de 35 °C ou moins et la température minimale de formation de film (MFFT) étant déterminée selon la norme DIN ISO 2115.

9. Polymère selon l'une quelconque des revendications 1 à 8, la température de transition vitreuse de point milieu (*T*_{mg}) étant dans la plage de 0 à 50 °C et la température de transition vitreuse de point milieu (*T*_{mg}) du polymère étant déterminée par une mesure de DSC selon la norme DIN EN ISO 11357-2.

10. Polymère selon l'une quelconque des revendications 1 à 9, le poids moléculaire moyen en poids Mw étant de 10 000 à 200 000 Daltons et le poids moléculaire moyen en poids étant déterminé par Chromatographie à Perméation de Gel selon la norme DIN 55672-1.

11. Forme de dosage, comprenant un noyau, comprenant un ingrédient biologiquement actif, et une couche de revêtement sur le noyau, la couche de revêtement étant composée d'un polymère selon l'une ou plusieurs des revendications 1 à 10 ou d'un polymère, polymérisé à partir d'un mélange de monomères comprenant les monomères
(a) 70 à 95 % en poids d'un ester d'alkyle en C₁ à C₁₂ d'acide acrylique ou d'acide méthacrylique,
(b) 2,5 à 25 % en poids d'un ester d'hydroxyalkyle en C₂ à C₆ d'acide acrylique ou d'acide méthacrylique,
(c) 2,5 à 15 % en poids d'un ester d'alkyle en C₂ à C₈ d'acide acrylique ou d'acide méthacrylique comportant un groupe cationique quaternaire dans le groupe alkyle.

12. Forme de dosage, selon la revendication 11, la couche de revêtement comprenant un polymère selon l'une ou plusieurs des revendications 1 à 10.

13. Forme de dosage selon la revendication 11 ou 12, la couche de revêtement ne contenant pas un plastifiant ou moins de 5 % en poids calculé sur le poids du polymère.

14. Utilisation de la forme de dosage selon l'une ou plusieurs des revendications 11 à 13 comme médicament administré par voie orale avec résistance contre l'influence d'éthanol.

15. Procédé pour la préparation d'un polymère selon l'une quelconque des revendications 1 à 10 à partir d'un mélange de monomères en la présence d'un initiateur de polymérisation et éventuellement d'un agent de transfert de chaînes par polymérisation en masse, polymérisation en suspension ou polymérisation en émulsion.

16. Utilisation d'un polymère, polymérisé à partir d'un mélange de monomères comprenant les monomères
(a) 70 à 95 % en poids d'un ester d'alkyle en C₁ à C₁₂ d'acide acrylique ou d'acide méthacrylique,
(b) 2,5 à 25 % en poids d'un ester d'hydroxyalkyle en C₂ à C₆ d'acide acrylique ou d'acide méthacrylique,
(c) 2,5 à 15 % en poids d'un ester d'alkyle en C₂ à C₈ d'acide acrylique ou d'acide méthacrylique comportant un groupe cationique quaternaire dans le groupe alkyle,
pour la préparation d'une forme de dosage selon l'une ou plusieurs des revendications 10 à 13 dotée d'un profil de libération prolongée et d'une résistance contre l'influence d'éthanol.

17. Utilisation selon la revendication 16, comprenant un polymère, polymérisé à partir d'un mélange de monomères comprenant les monomères
(a) 70 à 95 % en poids d'un ester d'alkyle en C₁ à C₁₂ d'acide acrylique ou d'acide méthacrylique,
(b) 2,5 à 15 % en poids d'un ou plusieurs esters d'hydroxyalkyle en C₂ à C₆ d'acide acrylique ou d'acide méthacrylique, et
(c) 2,5 à 15 % en poids d'un ou plusieurs esters d'alkyle en C₂ à C₈ d'acide acrylique ou d'acide méthacrylique comportant un groupe cationique quaternaire dans le groupe alkyle.
